# EUROPEAN PATENT APPLICATION

(11) **EP 1 741 444 A1**
(43) Date of publication of application: **10.01.2007**
(21) Application number: 05014581.2
(22) Date of filing: 05.07.2005
(51) Int. Cl.: A61K 45/00, A61K 38/08, A61P 13/10

(54) **Kinin antagonists for treating bladder dysfunction**

(71) Applicant: Jerini AG, 10115 Berlin (DE)
(72) Inventor: Gibson, Christoph, Dr., 10437 Berlin (DE); Hummel, Gerd, Dr., 13357 Berlin (DE); Reineke, Ulrich, Dr., 10249 Berlin (DE); Tradler, Thomas, Dr., 12439 Berlin (DE); Knolle, Jochen Dr., 10115 Berlin (DE)
(74) Representative: Bohmann, Armin K.

(57) **Abstract**

The present invention is related to the use of a kinin receptor antagonist for the manufacture of a medicament for the treatment and/or prevention of bladder dysfunction, whereby the kinin receptor is selected from the group comprising B1 and B2 receptors.

## Description

The invention relates to methods of using kinin antagonists for treating and/or alleviating the disease patterns associated with bladder dysfunction.

Bladder dysfunction is caused by a variety of disorders, preferably by disorders of the lower urinary tract. Bladder dysfunction affects the quality life of millions of men and women. In the United States as estimated 14.8 million patients suffer from urinary incontinence due to bladder dysfunction. The incidence of urinary incontinence increases with age of patients. Thus, the study of medical, epidemiological, and social aspects of aging (MESA) reported about 35% of randomly selected women aged >60 years as incontinent (Chaliha & Khullar, Urology. 2004 Mar;63 (3 Suppl 1):51-7).

Bladder dysfunction can be due to several disorders which are described in the following section but are not limited to these disorders.

Bladder dysfunction can be caused by neoplastic disorders, preferably by benign prostatic hyperplasia. In about 45% of men with BPH detrusor instability occurs and causes frequency, urgency, and urination during the night (Knutson et al., Neurourol Urodyn. 2001;20(3):237-47). Often, the prostate gland becomes enlarged as a man ages. This condition is called benign prostatic hyperplasia (BPH), or benign prostatic hypertrophy. As the prostate enlarges, the layer of surrounding tissue stops it from expanding, causing the gland to press against the urethra. The bladder wall becomes thicker and irritable and the bladder begins to contract even when it contains small amounts of urine, causing more frequent urination. Eventually, the bladder weakens and loses the ability to empty itself. Urine remains in the bladder. The irritative and obstructive symptoms of BPH, based on the narrowing of the urethra and partial emptying of the bladder cause many of the problems/symptoms associated with BPH like urinary urgency and frequency, nocturia and urinary incontinence as well as reduced urinary force and speed of flow. The occurrence of BPH increases with life expectancy. In 2000, there were 4.5 million visits to a physician for BPH in the United States (National Kidney And Urologic Diseases Information Clearinghouse, NIH-Publ.-No. 04-3012, February 2004).

Bladder dysfunction can also be due to instability of the detrusor. Detrusor instability is characterised by spontaneous and uninhibited contraction of the detrusor muscle during bladder filling, which means the pressure inside the bladder rises suddenly. Symptoms typical for detrusor instability are frequency, nocturia, urgency, urge incontinence, and nocturnal enuresis. The reason for detrusor instability can be either neurogenic, actually not known (idiopathic detrusor overactivity), myogenic or associated with bladder outlet obstruction (Bulmer & Abrams, Urol Int. 2004;72(1):1-12). Damage or dysfunction of the central nervous system like stroke, Parkinson's disease, multiple sclerosis and lesions of the spinal cord can lead to bladder dysfunction associated with bladder hyperreflexia (neurogenic Detrusor Overactivity, neurogenic detrusor instability, Moore & Gray, Nurs Res. 2004 Nov-Dec;53(6 Suppl):S36-41). The term "idiopathic detrusor overactivity" is used where the patient has no overt neurologic abnormality distinguishing this phenomenon from neurogenic detrusor overactivity (Bulmer & Abrams, Urol Int. 2004;72(1):1-12). Often, the likely explanation for detrusor instability in patients is multifactorial rather than a single underlying pathological process.

Furthermore, bladder dysfunction can be caused by bladder obstruction due to injury, surgery and anatomical abnormalities, neurological injuries, hormone dysregulation, diabetes or metabolic diseases (Moore & Gray, Nurs Res. 2004 Nov-Dec;53(6 Suppl):S36-41, Moghaddas et al., Menopause. 2005 May-Jun;12(3):318-24).

Apart from the above referenced pathologies, also bladder dysfunctions without proven pathology are oberserved, which is also referred to as idiopathic disorders of the lower urinary tract (Abrams P., Urology. 2003 Nov;62(5 Suppl 2):28-37; discussion 40-2). Independent from the various pathologies, the occurrence of many of these causes for bladder dysfunction increases with aging (Yoshida rt al., Urology. 2004 Mar;63(3 Suppl 1):17-23).

Currently, bladder dysfunction is treated by electrical stimulation, bladder or pelvic plexus training, surgery such as external sphincterotomy or removal of detrusor obstruction, dietary regulation, and medication. Medications especially in the field of overactive bladder which are marketed or under development, are cholinergic antagonists, adrenergic antagonists or agonists, vasopressin agonists, neurokinin antagonists, potassium channel activators, analgesics, NO donors, Ca²⁺ modulators, spasmolytics, smooth muscle modulators, preferably botulinus toxin, 5HT re-uptake inhibitors, purinergic receptor antagonists, PDE inhibitors, and/or VR1 modulators. Today's primary medications are anticholinergics. However, a significant percentage of patients do not respond to these medications or describe only partial symptom relief. For example 36% of women with urinary incontinence treated with Yentreve® (Duloxetin, Eli Lilly and Boehringer Ingelheim) did not respond to the treatment.

In addition, several severe side effects are reported by many patients. Widely reported are dry mouth, dry eyes, dry vagina, palpitations, drowsiness, constipation, blurred vision, and urinary retention or increased post-voiding residual volume upon chronic treatment. A subgroup of the anticholinergics which have a reduced pattern of side effects, consists of antimuscarinic compounds some of them being specific for the M₂ and M₃ receptor subtypes or selective against M₂ and/or M₅. However, many of the side effects especially dry mouth are still reported by many patients.

In view of these limitations of the means of the prior art for the treatment of bladder dysfunction, there is a need to provide means for the treatment of bladder dysfunction which are highly efficient. Furthermore, there is a need for means for the treatment of bladder dysfunction which have low side effects. There is also a need for means for the treatment of bladder dysfunction with high patient compliance. These various needs are in summary referred to herein as the problem under lying the present invention.

The means and their use disclosed herein are suitable to meet at least one of these needs. Insofar, the problem underlying the present invention are solved in a first aspect by

The problem underlying the present invention is solved in a first aspect by the use of a kinin receptor antagonist for the manufacture of a medicament for the treatment and/or prevention of bladder dysfunction, whereby the kinin receptor is selected from the group comprising B 1 and B2 receptors.

In an embodiment the kinin receptor is the B 1 receptor.

In a preferred embodiment the kinin receptor antagonist is preferably a B 1 receptor antagonist and is selected from the group comprising
Ac-Lys-Arg-Pro-Pro-Gly-Phe-Ser-D-Nal-Ile-OH (SEQ. ID NO. 1),
Ac-Lys-Arg-Pro-Pro-Gly-N-MePhe-Ser-D-Nal-Ile-OH (SEQ. ID NO. 2),
Ac-Lys-Lys-Arg-Pro-Pro-Gly-NMePhe-Ser-D-Nal-Ile-OH (SEQ. ID NO. 3),
Ac-Orn-Arg-Oic-Pro-Gly-NMePhe-Ser-D-Nal-Phe-OH (SEQ. ID NO. 4),
H-Lys-Lys-Arg-Pro-Hyp-Gly-Igl-Ser-D-Igl-Oic-OH (SEQ. ID NO. 5),
H-Lys-Lys-Arg-Pro-Hyp-Gly-Cpg-Ser-D-Tic-Cpg-OH (SEQ. ID NO. 6),
2-[1-(3,4-Dichloro-benzene sulfonyl)-3-oxo-1,2,3,4-tetrahydro-chinoxaline-2-yl]-N-{2-[4-(4,5-dihydro-1H-imidazole-2-yl)-phenyl]-ethyl}-acetamide,
N- {2-[4-(4,5-Dihydro-1H-imidazole-2-yl)-phenyl]-ethyl}-2-[1-(naphthaline-2-sulfonyl)-3-oxo-1,2,3,4-tetrahydro-chinoxaline-2-yl]-acetamide,
3-(3,4-Dichloro-phenyl)-N- {1-[4-(4,5-dihydro-1H-imidazole-2-yl)-benzyl]-2-oxo-2-pyrrolidine-1-yl-ethyl}-3-(naphthaline-2-sulfonyl amino)-propionamide,
4'-(1-{3-[(2,2-Difluoro-cyclopropane carbonyl)-amino]-4-methyl-pyridine-2-ylamino}-ethyl)-5-methyl-biphenyl-2-carboxylic acid methyl ester,
N-(4-Chloro-2-{1-[3'-fluoro-2'-(3-methyl-[1,2,4]oxadiazole-5-yl)-biphenyl-4-yl]-ethylamino}-pyridine-3-yl)-3,3,3-trifluoro propionamide,
3-Benzo[1,3]dioxol-5-yl-N-[2-[4-(2,6-dimethyl-piperidine-1-yl methyl)-phenyl]-1-(isopropyl-methyl-carbamoyl)-ethyl]-3-(6-methoxy-naphthaline-2-sulfonylamino)-propionamide,
{2-(2,2-Diphenyl-ethylamino)-5-[4-(4-isopropyl-piperazine-1-carbonyl)-piperidine-1-sulfonyl]-phenyl}-morpholine-4-yl-methanone, and
{2-(2,2-Diphenyl-ethylamino)-5-[4-(4-methyl-piperazine-1-carbonyl)-piperidine-1-sulfonyl]-phenyl}-morpholine-4-yl-methanone.

In an embodiment the kinin receptor is the B2 receptor.

In a preferred embodiment the kinin receptor antagonist is preferably a B2 receptor antagonist and is selected from the group comprising
MEN 11270,
H-D-Arg-Arg-Pro-Hyp-Gly-Igl-Ser-D-F5F-Igl-Arg-H (SEQ. ID NO. 7),
H-Arg-Arg-Pro-Hyp-Gly-Igl-Ser-D-Igl-Oic-Arg-OH (SEQ. ID NO. 8),
[H-D-Arg-Arg-Pro-Hyp-Gly-Phe-Cys-D-Phe-Leu-Arg-H]₂BSH (SEQ. ID NO. 9),
4- {2-[({[3-(3-Brom-2-methyl-imidazo[1,2-a]pyridine-8-yl oxymethyl)-2,4-dichlore-phenyl]-methyl-carbamoyl}-methyl)-carbamoyl]-vinyl}-N,N-dimethyl-benzamide,
3-(6-Acetylamino-pyridine-3-yl)-N-({[2,4-dichloro-3-(2-methyl-chinoline-8-yl oxymethyl)-phenyl]-methyl-carbamoyl}-methyl)-acrylamide,
1-[2,4-Dichloro-3-(2,4-dimethyl-chinoline-8-yl oxymethyl)-benzene sulfonyl]-pyrrolidine-2-carboxylic acid [3-(4-carbamimidoyl-benzoylamino)-propyl]-amide,
Bradizide,
4-(4-{1-[2,4-Dichloro-3-(2,4-dimethyl-chinoline-8-yl oxymethyl)-benzene sulfonyl]-pyrrolidine-2-carbonyl}-piperazine-1-carbonyl)-benzamidine, and
2-[5-(4-Cyano-benzoyl)-1-methyl-1H-pyrrol-2-yl]-N-[2,4-dichloro-3-(2-methyl-chinoline-8-yloxymethyl)-phenyl]-N-methyl-acetamide.

In an embodiment the kinin receptor antagonist is preferably a B2 receptor antagonist and is a peptide of the formula (I)

**Z-P-A-B-C-E-F-K-(D)Q-G-M-F'-I** (I)

in which:
- **Z**: is a₁) hydrogen, (C₁-C₈)-alkyl, (C₁-C₈)-alkanoyl, (C₁-C₈)-alkoxycarbonyl, (C₃-C₈)-cycloalkyl, (C₄-C₉)-cycloalkanoyl or (C₁-C₈)-alkylsulfonyl,
in which 1, 2 or 3 hydrogen atoms in each case are optionally individually and independent from each other replaced by 1, 2 or 3 identical or different radicals selected from the group consisting of carboxyl, NHR (1), [(C₁-C₄)-alkyl]NR(1) or [(C₆-C₁₀)-aryl-(C₁-C₄)-alkyl]NR(1), where R(1) is hydrogen or a urethane protective group, (C₁-C₄)-alkyl, (C₁-C₈)-alkylamino, (C₆-C₁₀)-aryl-(C₁-C₄)-alkylamino, hydroxyl, (C₁-C₄)-alkoxy, halogen, di-(C₁-C₈)-alkylamino, di-[(C₆-C₁₀)-aryl-(C₁-C₄)]-alkylamino, carbamoyl, phthalimido, 1,8-naphthalimido, sulfamoyl, (C₁-C₄) alkoxycarbonyl, (C₆-C₁₄)-aryl and (C₆-C₁₄)-aryl-(C₁-C₅)-alkyl,
or in which 1 hydrogen atom in each case is optionally replaced by a radical selected from the group consisting of (C₃-C₈)-cycloalkyl, (C₁-C₆)-alkylsulfonyl, (C₁-C₆)-alkylsulfinyl, (C₆-C₁₄)-aryl-(C₁-C₄)-alkylsulfonyl, (C₆-C₁₄)-aryl-(C₁-C₄)-alkylsulfinyl, (C₆-C₁₄)-aryl, (C₆-C₁₄)-aryloxy, (C₃-C₁₃)-heteroaryl and (C₃-C₁₃)- heteroaryloxy,
and 1 or 2 hydrogen atoms are replaced by 1 or 2 identical or different radicals selected from the group consisting of carboxyl, amino, (C₁-C₈)-alkylamino, hydroxyl, (C₁-C₄)-alkoxy, halogen, di-(C₁-C₈)-alkylamino, carbamoyl, sulfamoyl, (C₁-C₄)-alkoxycarbonyl, (C₆-C₁₄)-aryl and (C₆-C₁₄)-aryl-(C₁-C₅)-alkyl;
a₂) (C₆-C₁₄)-aryl, (C₇-C₁₅)-aroyl, (C₆-C₁₄)-arylsulfonyl, (C₃-C₁₃)-heteroaryl or (C₃-C₁₃)-heteroaroyl; or
a₃) carbamoyl which can optionally be substituted on the nitrogen by (C₁-C₈)-alkyl, (C₆-C₁₄)-aryl or (C₆-C₁₄)-aryl-(C₁-C₅)-alkyl;
where in the radicals defined under a₁), a₂) and a₃) the aryl, heteroaryl, aroyl, arylsulfonyl and heteroaroyl groups are optionally substituted by 1, 2, 3 or 4 radicals individually and independently selected from the group consisting of carboxyl, amino, nitro, (C₁-C₈)-alkylamino, hydroxyl, (C₁-C₆)-alkyl, (C₁-C₆)-alkoxy, (C₆-C₁₄)-aryl, (C₇-C₁₅)-aroyl, halogen, cyano, di-(C₁-C₈)-alkylamino, carbamoyl, sulfamoyl and (C₁-C₆)-alkoxycarbonyl;
- **P**: is a covalent bond or a radical of the formula II,

-NR(2)―(U)―CO- (II)

in which
R(2) is hydrogen, methyl or a urethane protective group,
U is (C₃-C₈)-cycloalkylidene, (C₆-C₁₄)-arylidene, (C₃-C₁₃)-heteroarylidene, (C₆-C₁₄)-aryl-(C₁-C₆)-alkylidene, which optionally individually and independent from each other can be substituted, or [CHR(3)]ₙ,
where n is any integer from 1-8, preferably any integer from 1-6,
any R(3) is independently and individually selected from the group comprising hydrogen, (C₁-C₆)-alkyl, (C₃-C₈)-cycloalkyl, (C₆-C₁₄)-aryl, (C₃-C₁₃)-heteroaryl, whereby under the proviso that R(3) is different from hydrogen, (C₁-C₆)-alkyl, (C₃-C₈)-cycloalkyl, (C₆-C₁₄)-aryl and (C₃-C₁₃)-heteroaryl are optionally monosubstituted by amino, substituted amino, amidino, substituted amidino, hydroxyl, carboxyl, carbamoyl, guanidino, substituted guanidino, ureido, substituted ureido, mercapto, methylmercapto, phenyl, 4-chlorophenyl, 4-fluorophenyl, 4-nitrophenyl, 4-methoxyphenyl, 4-hydroxyphenyl, phthalimido, 1,8-naphthalimido, 4-imidazolyl, 3-indolyl, 2-thienyl, 3-thienyl, 2-pyridyl, 3-pyridyl or cyclohexyl,
or
in which R(2) and R(3), together with the atoms carrying them, form a mono-, bi- or tricyclic ring system having 2 to 15 carbon atoms;
- **A**: is defined as P;
- **B**: is a basic amino acid in the L-or D-configuration, which can be substituted in the side chain;
- **C**: is a compound of the formula III a or III b 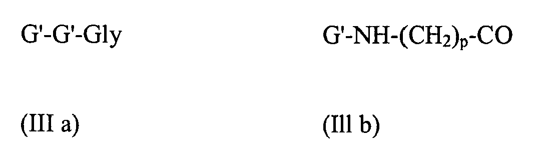 in which
p is any integer from 2 to 8, and
any G' is independently a radical of the formula IV

-NR(4)-CHR(5)-CO- (IV)

in which
R(4) and R(5), together with the atoms carrying them, form a heterocyclic mono-, bi-or tricyclic ring system having 2 to 15 carbon atoms;
- **E**: is a radical of a neutral, acidic or basic, aliphatic or alicyclic-aliphatic amino acid;
- **F**: independently of one another is a radical of a neutral, acidic or basic, aliphatic or aromatic amino acid which can be substituted in the side chain, or a covalent bond;
- **(D)Q**: is D-Tic, D-Phe, D-Oic, D-Thi or D-Nal, any of which is optionally substituted by halogen, methyl or methoxy or is a radical of the formula (V) below 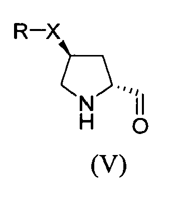 in which
X is oxygen, sulfur or a covalent bond;
R is hydrogen, (C₁-C₈)-alkyl, (C₃-C₈)-cycloalkyl, (C₆-C₁₄)-aryl, (C₆-C₁₄)-aryl-(C₁-C₄)-alkyl, where the alicyclic system can optionally be substituted by halogen, methyl or methoxy;
- **G**: is defined as G' above or is a covalent bond;
- **F'**: is defined as F, is a radical -NH-(CH₂)_{q}-, where q=2 to 8, or, if G is not a direct bond, is a direct bond;
- **I**: is -OH, -NH₂ or NHC₂H₅;
- **K**: is the radical -NH-(CH₂)ₓ-CO-, where x=1-4, or is a covalent bond, and
- **M**: is defined as F,
or a physiologically tolerable salt thereof.

In a preferred embodiment the peptide is a peptide of formula I wherein
- **Z**: is as defined above under a₁₎, a₂) or a₃₎, preferably hydrogen
- **P**: is a covalent bond or a radical of the formula II

-NR(2)-(U)―CO― (II)

where
U is CHR(3),
R(3) is as defined above, and
R(2) is H or CH₃,
- **A**: is a covalent bond.
or a physiologically tolerable salt thereof.

In a more preferred embodiment which is preferably an embodiment of the preceding embodiment
- **Z**: is as defined above under a₁), a₂) or a₃), preferably hydrogen
- **P**: is a covalent bond or a radical of the formula II

-NR(2)―(U)-CO- (II)

where
U is CHR(3) and
R(3) is individually and independently selected from the group comprising hydrogen, (C1-C6)-alkyl, (C3-C8)-cycloalkyl, (C6-C14)-aryl, (C3-C13)-heteroaryl, whereby under the proviso that R(3) is different from hydrogen, (C₁-C₆)-alkyl, (C₃-C₈)-cycloalkyl, (C₆-C₁₄)-aryl and (C₃-C₁₃)-heteroaryl are optionally monosubstituted by amino, substituted amino, hydroxyl, carboxyl, carbamoyl, guanidino, substituted guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pyridyl or cyclohexyl,
or in which R(2) and R(3), together with the atoms carrying them, form a mono-, bi-or tricyclic ring system having 2 to 15 carbon atoms,
R(2) is H or CH3;
- **A**: is a covalent bond;
- **(D)Q**: is D-Tic.

In an even more preferred embodiment the peptide is
H-D-Arg-Arg-Pro-Hyp-Gly-Thi-Ser-D-Tic-Oic-Arg-OH (SEQ. ID NO. 10), para-guanidobenzoyl-Arg-Pro-Hyp-Gly-Thi-Ser-D-Tic-Oic-Arg-OH (SEQ. ID NO. 11),
H-D-Arg-Arg-Pro-Hyp-Gly-Phe-Ser-D-HypE(transpropyl)-Oic-Arg-OH (SEQ. ID NO. 12),
H-D-Arg-Arg-Pro-Hyp-Gly-Cpg-Ser-D-Cpg-Cpg-Arg-OH (SEQ. ID NO. 13),
H-D-Arg-Arg-Pro-Pro-Gly-Thi-Ser-D-Tic-Oic-Arg-OH (SEQ. ID NO. 14),
H-Arg(Tos)-Pro-Hyp-Gly-Thi-Ser-D-Tic-Oic-Arg-OH (SEQ. ID NO. 15),
H-Arg(Tos)-Pro-Hyp-Gly-Phe-Ser-D-Tic-Oic-Arg-OH (SEQ. ID NO. 16),
H-D-Arg -Arg-Pro-Hyp-Gly-Phe-Ser-D-Tic-Oic-Arg-OH (SEQ. ID NO. 17),
Fmoc-D-Arg-Arg-Pro-Hyp-Gly-Thi-Ser-D-Tic-Oic-Arg-OH (SEQ. ID NO. 18),
Fmoc-Aoc-D-Arg-Arg-Pro-Hyp-GIy-Thi-Ser-D-Tic-Oic-Arg-OH (SEQ. ID NO. 19),
Fmoc-ε-aminocaproyl-D-Arg-Arg-Pro-Hyp-Gly-Thi-Ser-D-Tic-Oic-Arg-OH (SEQ. ID NO. 20),
benzoyl-D-Arg-Arg-Pro-Hyp-Gly-Thi-Ser-D-Tic-Oic-Arg-OH (SEQ. ID NO. 21),
cyclohexylcarbonyl-D-Arg-Arg-Pro-Hyp-Gly-Thi-Ser-D-Tic-Oic-Arg-OH (SEQ. ID NO. 22),
Fmoc-Aeg(Fmoc)-D-Arg-Arg-Pro-Hyp-Gly-Thi-Ser-D-Tic-Oic-Arg-OH (SEQ. ID NO. 23),
Fmoc-Aeg(Fmoc)-Arg-Pro-Hyp-Gly-Thi-Ser-D-Tic-Oic-Arg-OH (SEQ. ID NO. 24),
indol-3-yl-acetyl-D-Arg-Arg-Pro-Hyp-Gly-Thi-Ser-D-Tic-Oic-Arg-OH (SEQ. ID NO. 25),
dibenzylacetyl-D-Arg-Arg-Pro-Hyp-Gly-Thi-Ser-D-Tic-Oic-Arg-OH (SEQ. ID NO. 26),
or a physiologically tolerable salt thereof.

In a particularly preferred embodiment the peptide is
H-D-Arg-Arg-Pro-Hyp-Gly-Thi-Ser-D-Tic-Oic-Arg-OH (SEQ. ID NO. 10) or
para-guanidobenzoyl-Arg-Pro-Hyp-Gly-Thi-Ser-D-Tic-Oic-Arg-OH (SEQ. ID NO.11); preferably
H-D-Arg-Arg-Pro-Hyp-Gly-Thi-Ser-D-Tic-Oic-Arg-OH (HOE 140) (SEQ. ID NO. 10)
or a physiologically tolerated salt thereof.

In an embodiment the bladder dysfunction is associated with one or more disease patterns selected from the group comprising urinary frequency, urinary urgency, dysuria, urinary incontinence, enuresis, loss of bladder function, and nocturia.

In a preferred embodiment the bladder dysfunction is associated with urinary frequency.

In a further preferred embodiment the bladder dysfunction is associated with urinary urgency.

In a still further preferred embodiment the bladder dysfunction is associated with urinary incontinence.

In an embodiment the bladder dysfunction is correlated with and/or caused by one or more disorders selected from the group comprising neurogenic, myogenic, neoplastic, preferably benign prostatic hyperplasia, inflammatory, metabolic and idiopathic disorders, prostate hypertrophy, hormone dysregulation, bladder obstruction due to surgery or injury, anatomical changes of the urogenital tract, multiple sclerosis, parkinson's disease, stroke, diabetes and aging.

In a preferred embodiment the bladder dysfunction is correlated with and/or caused by neurogenic disorders.

In a further preferred embodiment the bladder dysfunction is correlated with and/or caused by myogenic and/or inflammatory disorders.

In a still further preferred embodiment the bladder dysfunction is correlated with and/or caused by idiopathic disorders.

In another preferred embodiment the bladder dysfunction is correlated with and/or caused by neoplastic and/or metabolic disorders and/or is associated with diabetes or aging.

In still another preferred embodiment the bladder dysfunction is correlated with C-fibre activation and/or sensitization, preferably pathological C-fibre activation and/or sensitization.

In an embodiment the bladder dysfunction and the correlated and/or causing disease patterns cannot be treated or prevented by a compound selected from the group comprising cholinergic antagonists, adrenergic antagonists, adrenergic agonists, vasopressin agonists, neurokinin antagonists, potassium channel activators, analgesics, NO donors, Ca²⁺ modulators, spasmolytics, muscle relaxants, preferably botulinus toxin, 5HT re-uptake inhibitors, purinergic receptor antagonists, PDE inhibitors, and VR1 modulators.

In an embodiment the medicament is to be administered to a patient in need thereof in a therapeutically effective amount.

In an embodiment the medicament is present in an oral dosage form, whereby such oral dosage form is selected from the group comprising powder, preferably a dispersible powder, capsule, tablet, solution, and liquid suspension.

In an embodiment the medicament is for parenteral administration

In an embodiment the medicament is for local and/or systemic administration.

In an embodiment the medicament is for administration to the patient by a route selected from the group comprising intravenous, subcutaneous, intravesical, intramuscular, intrathecal, nasal, rectal, sublingual, transurethral, intravaginal perivaginal, intraperitoneal, transmucosal, transdermal administration and inhalation.

In an embodiment the medicament comprises at least one further pharmaceutically active compound.

In a preferred embodiment the further pharmaceutically active compound is selected from the group comprising cholinergic antagonists, adrenergic antagonists or agonists, vasopressin agonists, neurokinin antagonists, potassium channel activators, analgesics, NO donors, Ca²⁺ modulators, spasmolytics, muscle relaxants, preferably botulinus toxin, 5HT re-uptake inhibitors, purinergic receptor antagonists, PDE inhibitors, and VR1 modulators.

In an embodiment the kinin antagonist and/or at least one further pharmaceutically active compound is/are present as a pharmaceutically acceptable salt, ester, amides, prodrug, or a pharmaceutically active solvate.

In an embodiment the kinin antagonist is metabolized to one or more pharmaceutically active molecules.

In an embodiment the medicament comprises a pharmaceutically acceptable carrier, diluent or excipient.

In an embodiment the medicament comprises a multitude of individualised dosages and/or administration forms.

In an embodiment the medicament is used for the treatment and/or prevention of bladder dysfunction in animals.

The present inventors have surprisingly found that kinin receptor antagonists can be used for the treatment of bladder dysfunction. This surprising finding can be explained by the fact that kinin receptors are constitutively expressed on C-fibres (Steranka et al., Proc Natl Acad Sci U S A. 1988 May;85(9):3245-9) which in turn are involved in various mechanisms related to or associated with bladder dysfunction.

There are basically two kinin receptors which are involved in the broader sense in bladder dysfunction, namely the kinin B 1 receptor and the kinin B2 receptor. Due to the involvement of both receptors, antagonists of both receptors can be used for the treatment of bladder dysfunction. The kinin B1 receptor is, among others, described in Leeb-Lundberg et al., Pharmacol Rev. 2005 Mar;57(1):27-77. The kinin B2 receptor is, among others, described in Leeb-Lundberg et al., Pharmacol Rev. 2005 Mar;57(1):27-77.

The term "kinin antagonist" is preferably used in its conventional sense to refer to a compound that binds to and antagonizes kinin receptors. Unless otherwise indicated, the term "kinin antagonist" is intended to include B1 receptor antagonists and B2 receptor antagonists as in particular disclosed further herein, as well as acids, salts, esters, amides, prodrugs, active metabolites, and other derivatives thereof. Furthermore, it is understood that any salts, esters, amides, prodrugs, active metabolites or other derivatives thereof are pharmaceutically acceptable as well as pharmacologically active.

A kinin antagonist can be characterized as such in an appropriate assay. Such assay is known to the ones skilled in the art and, among others, described in Wieczorek et al., Biochem Pharmacol. 1997 Jul 15;54(2):283-91.

For example, in the functional calcium flux assay as described herein, B2 receptor activation triggers activation of phospholipase C and subsequent inositolphosphate release. This initiates the release of the second messenger calcium from stores in the endoplasmatic reticulum into the cytosol of the cell. Cytosolic calcium ions bind to cytosolic calcium dyes (e.g. Fura-2) and alter their fluorescent properties. In such a way, fluorescence properties of the cells correspond directly to the cytosolic calcium concentration. Therefore, after pre-incubation and loading of cells with the calcium dye, changes in the intracellular calcium concentration triggered by activation of the B2 kinin receptor can be monitored time-resolved with appropriate devices, e.g. fluorescence spectrophotometers and readers. In this type of assay, inhibition of agonist-induced receptor activation by co-incubation with kinin receptor antagonists will circumvent or lower the agonist-induced increase in the cytosolic calcium concentration.

In connection with the present invention, the following terms have preferably the following meaning if not indicated to the contrary.

By "lower urinary tract" is intended all parts of the urinary system except of the kidneys.

By "bladder dysfunction" is intended any pathological condition involving the urinary bladder.

The term "disease pattern" as used herein is defined as symptom or pathological situation associated with bladder dysfunction. Disease patterns comprise single symptoms or pathological situations as well as combinations of different symptoms or pathological situations. Disease patterns associated with bladder dysfunction can include, but are not limited to, urinary frequency, urinary urgency, dysuria, urinary incontinence, enuresis, loss of function, and nocturia, some of them summarized as "overactive bladder".

"Urinary urgency" is the acute urge to urinate with no or only a small success to delay the urination. Patients partially irrespective of the real urine volume in the bladder feel the acute urge to urinate.

If correlated with only a small success to delay urination, patients suffer from the disease pattern referred to as "urinary frequency" which means that the frequency of urination is higher then desired by patients. As there is considerable interpersonal variation in the number of times in a day that an individual would normally expect to urinate, "urination is higher than desired by patients" is further defined as a greater number of times per day than that patient's historical baseline. "Historical baseline"" is further defined as the median number of times the patient urinated per day during a normal or desirable time period.

If urinary urgency is correlated with no success to delay urination, patients suffer from "urinary incontinence".

By "urge incontinence" or "urinary urge incontinence" is intended the involuntary loss of urine associated with an abrupt and strong desire to void. By "stress incontinence" or "urinary stress incontinence" is intended a medical condition in which urine leaks when a person coughs, sneezes, laughs, exercises, lifts heavy objects, or does anything that puts pressure on the bladder.

In "dysuria", patients feel a painful desire to void together with aggravated micturition. Urinary incontinence means a state, in which unwanted loss of urine forms a social and hygienically relevant problem (definition of the International Continence Society).

By the term "enuresis" the unwanted micturition due to lacking control of micturition by children older than 4 years is designated.

"Loss of function" is the complete loss of bladder control by the patient, often caused by severe neurological lesions or diseases.

"Nocturia" describes the increased production of urine overnight, leading to more phases of awaking to urinate than wanted by the patient.

"Overactive bladder" as defined in the 2002 International Continence Society standardization as disease pattern comprises several symptoms, usually with or without urge incontinence and in most cases associated with frequency and nocturia. An important point for exclusion diagnosis of overactive bladder is the absence of infection or other proven pathology (Abrams et al., Report from the standardisation sub-committee of the Intl. Continence Soc., Urology, 61: 37, 2003).

It will be understood by the ones skilled in the art that, in principle, any form of bladder dysfunction can be treated using the kinin antagonists disclosed herein. Apart from the various forms of bladder dysfunction described herein elsewhere, a particular form of bladder dysfunction is prostatitis, prostadynia, and cystitis preferably interstitial cystitis.

A particularly preferred group of kinin antagonists which can be used in accordance with the present invention are B 1 receptor antagonists.

More preferred B 1 receptor antagonists are the following ones.
H-Ac-Lys-Arg-Pro-Pro-Gly-Phe-Ser-D- Nal-Ile-OH (R715),
Ac-Lys-Arg-Pro-Pro-Gly-N-MePhe-Ser-D-Nal-Ile-OH (R892), and
AcLys-Lys-Arg-Pro-Pro-Gly-NMePhe-Ser-D-Nal-Ile-OH (R914) as described in Gobeil, F., Jr.; Charland, S.; Filteau, C.; Perron, S. I.; Neugebauer, W.; Regoli, D. Hypertension 1999, 33, 823-9;
AcOrn-Arg-Oic-Pro-Gly-NMePhe-Ser-D-Nal-Phe-OH (R954), as described in Gabra, B. H.; Sirois, P. Peptides 2003, 24, 1131-9;
H-Lys-Lys-Arg-Pro-Hyp-Gly-Igl-Ser-D-Igl-Oic-OH (B9858) and
H-Lys-Lys-Arg-Pro-Hyp-Gly-Cpg-Ser-D-Tic-Cpg-OH (B9958)
as described in Stewart, J. M.; Gera, L.; Hanson, W.; Zuzack, J. S.; Burkard, M.; McCullough, R.; Whalley, E. T. Immunopharmacology 1996, 33, 51-60; 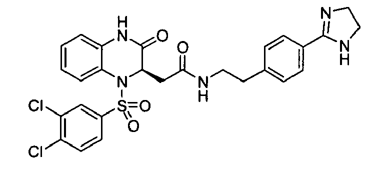 2-[1-(3,4-Dichloro-benzenesulfonyl)-3-oxo-1,2,3,4-tetrahydro-quinoxalin-2-yl]-N-{2-[4-(4,5-dihydro-1H-imidazol-2-yl)-phenyl]-ethyl}-acetamide, 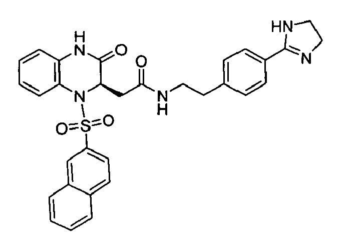 N- {2-[4-(4,5-Dihydro-1H-imidazol-2-yl)-phenyl]-ethyl}-2-[1-(naphthalene-2-sulfonyl)-3-oxo-1,2,3,4-tetrahydro-quinoxalin-2-yl]-acetamide, 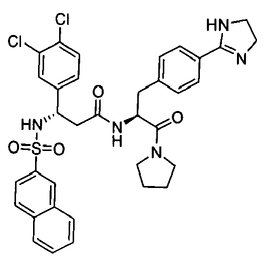 3-(3,4-Dichloro-phenyl)-N-{1-[4-(4,5-dihydro-1H-imidazol-2-yl)-benzyl]-2-oxo-2-pyrrolidin-1-yl-ethyl}-3-(naphthalene-2-sulfonylamino)-propionamide, 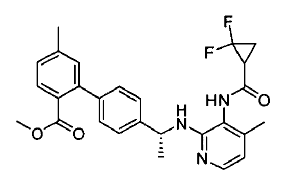 4'-(1- {3-[(2,2-Difluoro-cyclopropanecarbonyl)-amino]-4-methyl-pyridin-2-ylamino}-ethyl)-5-methyl-biphenyl-2-carboxylic acid methyl ester, 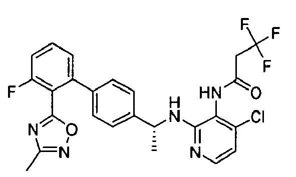 N-(4-Chloro-2-{1-[3'-fluoro-2'-(3-methyl-[1,2,4]oxadiazol-5-yl)-biphenyl-4-yl]-ethylamino}-pyridin-3-yl)-3,3,3-trifluoro-propionamide,
as described in Hess, J. F.; Ransom, R. W.; Zeng, Z.; Chang, R. S.; Hey, P. J.; Warren, L.; Harrell, C. M.; Murphy, K. L.; Chen, T. B.; Miller, P. J.; Lis, E.; Reiss, D.; Gibson, R. E.; Markowitz, M. K.; DiPardo, R. M.; Su, D. S.; Bock, M. G.; Gould, R. J.; Pettibone, D. J. J. Pharmacol. Exp. Ther. 2004, 310, 488-97; 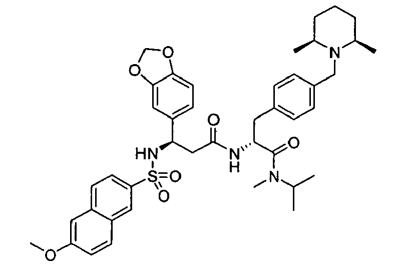 3-Benzo[1,3]dioxol-5-yl-N-[2-[4-(2,6-dimethyl-piperidin-1-ylmethyl)-phenyl]-1-(isopropyl-methyl-carbamoyl)-ethyl]-3-(6-methoxy-naphthalene-2-sulfonylamino)-propionamide, as described by Gougat, J.; Ferrari, B.; Sarran, L.; Planchenault, C.; Poncelet, M.; Maruani, J.; Alonso, R.; Cudennec, A.; Croci, T.; Guagnini, F.; Urban-Szabo, K.; Martinolle, J. P.; Soubrie, P.; Finance, O.; Le Fur, G. J. Pharmacol. Exp. Ther. 2004, 309, 661-9; and 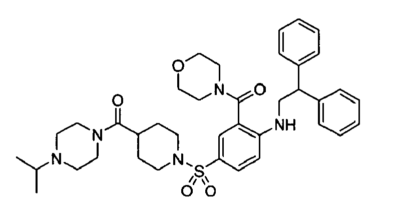 {2-(2,2-Diphenyl-ethylamino)-5-[4-(4-isopropyl-piperazine-1-carbonyl)-piperidine-1-sulfonyl]-phenyl}-morpholin-4-yl-methanone, 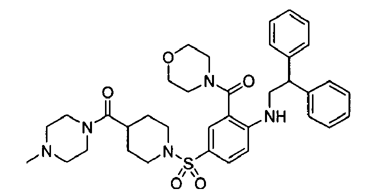 {2-(2,2-Diphenyl-ethylamino)-5-[4-(4-methyl-piperazine-1-carbonyl)-piperidine-1-sulfonyl]-phenyl}-morpholin-4-yl-methanone,
as described by Ritchie, T. J.; Dziadulewicz, E. K.; Culshaw, A. J.; Muller, W.; Burgess, G. M.; Bloomfield, G. C.; Drake, G. S.; Dunstan, A. R.; Beattie, D.; Hughes, G. A.; Ganju, P.; McIntyre, P.; Bevan, S. J.; Davis, C.; Yaqoob, M. J. Med. Chem. 2004, 47, 4642-4.

It will be acknowledged by the ones skilled in the art that although some of the above-mentioned compounds are categorized as B1 receptor antagonist and as B2 receptor antagonist, a B 1 receptor antagonist may also be effective as a B2 receptor antagonist, and a B2 receptor antagonist may also be effective as a B1 receptor antagonist. Preferably, the B2 receptor antagonist activity of a B1 receptor antagonist is significantly less pronounced than its B 1 receptor antagonist activity. Also preferably the B 1 receptor antagonist activity of a B2 receptor antagonist is significantly less pronounced than its B2 receptor antagonist activity. Even more preferably, the difference in activity is by a factor from 50 to 500 or more, preferably by a factor greater than 500.

A particularly preferred group of kinin antagonists which can be used in accordance with the present invention are B2 receptor antagonists.

More preferred B2 receptor antagonists are the following ones. 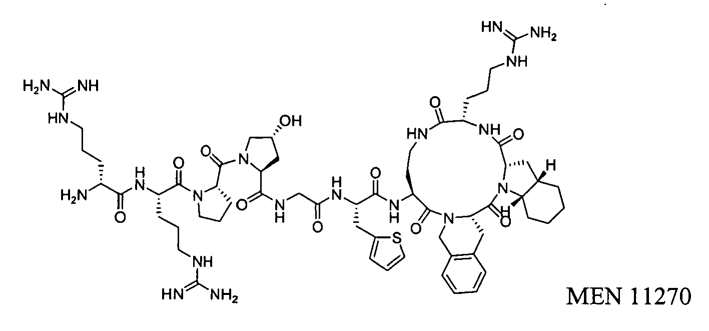 as described in Meini, S.; Quartara, L.; Rizzi, A.; Patacchini, R.; Cucchi, P.; Giolitti, A.; Calo, G.; Regoli, D.; Criscuoli, M.; Maggi, C. A. J. Pharmacol. Exp. Ther. 1999, 289, 1250-6;
H-D-Arg-Arg-Pro-Hyp-Gly-Igl-Ser-D-F5F-Igl-Arg-OH (B-10056), and H-Arg-Arg-Pro-Hyp-Gly-Igl-Ser-D-Igl-Oic-Arg-OH (B-9430) as described in Stewart, J. M.; Gera, L.; York, E. J.; Chan, D. C.; Bunn, P. Immunopharmacology 1999, 43, 155-61*;*
[H-D-Arg-Arg-Pro-Hyp-Gly-Phe-Cys-D-Phe-Leu-Arg-OH]₂BSH, with BSH=bissuccinimidohexane (CP-0127Bradycor) as described byHeitsch, H. Curr. Med. Chem. 2002, 9, 913-28 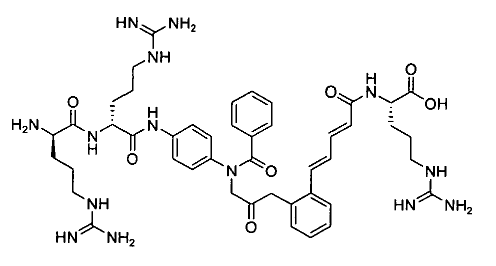 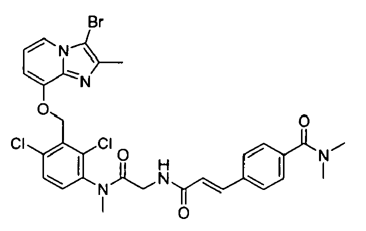 FR 167344 ; which is 4-{2-[({[3-(3-Bromo-2-methyl-imidazo[1,2-a]pyridin-8-yloxymethyl)-2,4-dichloro-phenyl]-methyl-carbamoyl}-methyl)-carbamoyl]-vinyl}-N,N-dimethyl-benzamide, 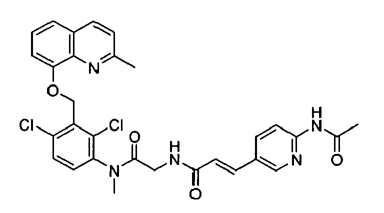 FR 173657 or FK3657 which is 3-(6-Acetylamino-pyridin-3-yl)-N-({[2,4-dichloro-3-(2-methyl-quinolin-8-yloxymethyl)-phenyl]-methyl-carbamoyl}-methyl)-acrylamide 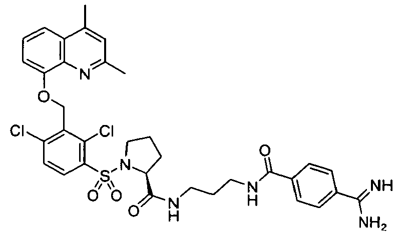 LF-160687 or Anatibant which is 1-[2,4-Dichloro-3-(2,4-dimethyl-quinolin-8-yloxymethyl)-benzenesulfonyl]-pyrrolidine-2-carboxylic acid [3-(4-carbamimidoyl-benzoylamino)-propyl]-amide, 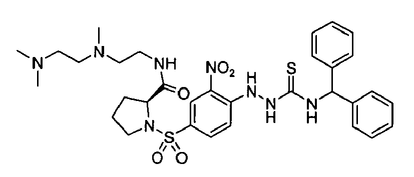 Bradizide, 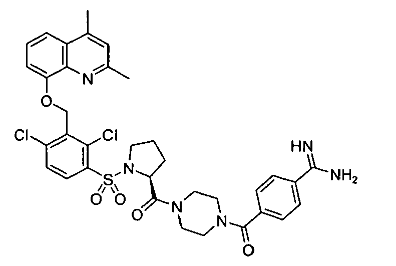 LF-160335; which is 4-(4-{1-[2,4-Dichloro-3-(2,4-dimethyl-quinolin-8-yloxymethyl)-benzenesulfonyl]-pyrrolidine-2-carbonyl}-piperazine-1-carbonyl)-benzamidine as decribed by Pruneau, D.; Luccarini, J. M.; Fouchet, C.; Defrene, E.; Franck, R. M.; Loillier, B.; Duclos, H.; Robert, C.; Cremers, B.; Belichard, P.; Paquet, J. L. Br. J. Pharmacol. 1998, 125, 365-72 ; 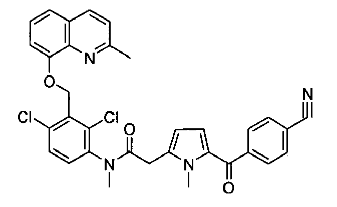 2-[5-(4-Cyano-benzoyl)-1-methyl-1H-pyrrol-2-yl]-N-[2,4-dichloro-3-(2-methyl-quinolin-8-yloxymethyl)-phenyl]-N-methyl-acetamide, as described by R., C. In Medicinal Chemistry - 28th National Symposium (Part II) - Overnight Report; IDdb meeting Report: San Diego, 2002.

A particularly preferred B2 receptor antagonist is Icatibant and its pharmaceutically active derivatives thereof. Icatibant and said derivatives are, among others, also described in US 0564833A.

In a lot of scientific publications it was shown, that Icatibant (which is also referred to as Hoe140 or JE049, Lembeck et al., Br J Pharmacol. 1991 Feb;102(2):297-304), is a highly efficient antagonist of kinin receptors of the B2 subtype. Icatibant inhibits the binding of bradykinin with an IC₅₀ of 1.07 nM on guinea pig ileum preparations (Hock et al., Br J Pharmacol. 1991 Mar;102(3):769-73). In isolated organ preparations of rat uterus, Icatibant inhibited bradykinin-induced contractions with IC₅₀ values of 4.9 nM (Hock et al., Br J Pharmacol. 1991 Mar;102(3):769-73) and with p*K_{B}* values of 8.4 on the human umbilical vein (Quartara et al., Eur J Med Chem. 2000 Nov;35(11):1001-10). Also, Icatibant efficiently inhibited BK-mediated inositol phosphate generation in human INT407 cells triggered by guinea pig B2 receptors (Robert et al., Br J Pharmacol. 2002 Jan;135(2):462-8).

It will be acknowledged by the ones skilled in the art that the use of kinin antagonists in accordance with the present invention may also comprise the administration of another pharmaceutically active agent or compound. Such another pharmaceutically active agent is preferably selected from the group comprising cholinergic antagonists, adrenergic antagonists, adrenergic agonists, vasopressin agonists, neurokinin antagonists, potassium channel activators, analgesics, NO donors, Ca²⁺ modulators, spasmolytics, muscle relaxants, preferably botulinus toxin, 5HT re-uptake inhibitors, purinergic receptor antagonists, PDE inhibitors, and VR1 modulators.

The term "cholinergic antagonist" as preferably used herein refers to any acetylcholine receptor antagonist, including antagonists of nicotinic and/or muscarinic acetylcholine receptors. The term "antagonist of nicotinic acetylcholine receptors" as used herein is intended any nicotinic acytylcholine receptor antagonist. The term "antagonist of muscarinic receptors" as used herein is intended as any muscarinic acetylcholine receptor antagonist. Unless otherwise indicated, the terms "cholinergic antagonist", "antagonist of nicotinic acetylcholine receptors" and "antagonist of muscarinic receptors" are intended to include cholinergic antagonists, antagonists of nicotinic acetylcholine receptors and antagonists of muscarinic receptors as disclosed further herein, as well as acids, salts, esters, amides, prodrugs, active metabolites, and other derivatives thereof. Further, it is understood that any salts, esters, amides, prodrugs, active metabolites or other derivatives thereof are pharmaceutically acceptable as well as pharmacologically active.

Acetylcholine is a chemical neurotransmitter in the nervous systems of all animals. "Cholinergic neurotransmission" refers to neurotransmission that involves acetylcholine, and has been implicated in the control of functions as diverse as locomotion, digestion, cardiac rate, "fight or flight" responses, and learning and memory (Salvaterra (February 2000) Acetylcholine. In Encyclopedia of Life Sciences. London: Nature Publishing Group, http:/www.els.net). Receptors for acetylcholine are classified into two general categories based on the plant alkaloids that preferentially interact with them: 1) nicotinic (nicotine binding); or 2) muscarinic (muscarine binding) (See, e.g., Salvaterra, Acetylcholine, supra).

The two general categories of acetylcholine receptors may be further divided into subclasses based upon differences in their pharmacological and electrophysiological properties. For example, nicotinic receptors are composed of a variety of subunits that are used to identify the following subclasses: 1) muscle nicotinic acetylcholine receptors; 2) neuronal nicotinic acetylcholine receptors that do not bind the snake venom α-bungarotoxin; and 3) neuronal nicotinic acetylcholine receptors that do bind the snake venom α-bungarotoxin (Dani et al. (July 1999) Nicotinic Acetylcholine Receptors in Neurons. In Encyclopedia of Life Sciences. London: Nature Publishing Group, http:/www.els.net; Lindstrom (October 2001) Nicotinic Acetylcholine Receptors. In Encyclopedia of Life Sciences. London: Nature Publishing Group, http:/www.els.net). By contrast, muscarinic receptors may be divided into five subclasses, labeled M₁-M₅, and preferentially couple with specific G-proteins (M₁, M₃, and M₅ with G_{q}; M₂ and M₄ with Gᵢ/Gₒ) (Nathanson (July 1999) Muscarinic Acetylcholine Receptors. In Encyclopedia of Life Sciences. London: Nature Publishing Group, http:/www.els.net). In general, muscarinic receptors have been implicated in bladder function (See, e.g., Appell (2002) Cleve. Clin. J. Med. 69: 761-9; Diouf et al. (2002) Bioorg. Med. Chem. Lett. 12: 2535-9; Crandall (2001) J. Womens Health Gend. Based Med. 10: 735-43; Chapple (2000) Urology 55: 33-46).

In view of this, agents useful in the present invention include any anticholinergic agent, specifically, any antimuscarinic agent. Particularly useful in the methods of the present invention is oxybutynin, also known as 4-diethylaminio-2-butynyl phenylcyclohexyglycolate. It has the following structure:

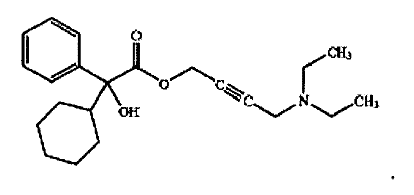

Ditropan^{®} (oxybutynin chloride) is the d,l racemic mixture of the above compound, which is known to exert antispasmodic effect on smooth muscle and inhibit the muscarinic action of acetylcholine on smooth muscle. Metabolites and isomers of oxybutynin have also been shown to have activity useful according to the present invention. Examples include, but are not limited to, N-desethyl-oxybutynin and S-oxybutynin (see, e.g., U.S. Pat. Nos. 5,736,577 and 5,532,278).

Additional compounds that have been identified as antimuscarinic agents and that are useful in the present invention include, but are not limited to:
a. Darifenacin (Daryon^{®}) or acids, salts, enantiomers, analogs, esters, amides, prodrugs, active metabolites, and derivatives thereof;
b. Solifenacin or acids, salts, enantiomers, analogs, esters, amides, prodrugs, active metabolites, and derivatives thereof,
c. YM-905 (solifenacin succinate) or acids, salts, enantiomers, analogs, esters, amides, prodrugs, active metabolites, and derivatives thereof;
d. Solifenacin monohydrochloride or acids, salts, enantiomers, analogs, esters, amides, prodrugs, active metabolites, and derivatives thereof;
e. Tolterodine (Detrol^{®}) or acids, salts, enantiomers, analogs, esters, amides, prodrugs, active metabolites, and derivatives thereof,
f. Propiverine (Detrunorm^{®}) or acids, salts, enantiomers, analogs, esters, amides, prodrugs, active metabolites, and derivatives thereof,
g. Propantheline bromide (Pro-Banthine^{®}or acids, salts, enantiomers, analogs, esters, amides, prodrugs, active metabolites, and derivatives thereof;
h. Hyoscyamine sulfate (Levsin^{®}Cystospaz^{®}or acids, salts, enantiomers, analogs, esters, amides, prodrugs, active metabolites, and derivatives thereof;
i. Dicyclomine hydrochloride (Bentyl^{®}or acids, salts, enantiomers, analogs, esters, amides, prodrugs, active metabolites, and derivatives thereof,
j. Flavoxate hydrochloride (Urispas^{®}or acids, salts, enantiomers, analogs, esters, amides, prodrugs, active metabolites, and derivatives thereof;
k. d,l (racemic) 4-diethylamino-2-butynyl phenylcyclohexylglycolate or acids, salts, enantiomers, analogs, esters, amides, prodrugs, active metabolites, and derivatives thereof;
1. (R)-N,N-diisopropyl-3-(2-hydroxy-5-methylphenyl)-3-phenylpropanamine L-hydrogen tartrate or acids, salts, enantiomers, analogs, esters, amides, prodrugs, active metabolites, and derivatives thereof;
m. (+)-(1S,3'R)-quinuclidin-3'-yl-1-phenyl-1,2,3,4-tetrahydroisoquinoline-2-carboxylate monosuccinate or acids, salts, enantiomers, analogs, esters, amides, prodrugs, active metabolites, and derivatives thereof;
n. alpha(+)-4-(Dimethylamino)-3-methyl-1,2-diphenyl-2-butanol proprionate or acids, salts, enantiomers, analogs, esters, amides, prodrugs, active metabolites, and derivatives thereof;
o. 1-methyl-4-piperidyl diphenylpropoxyacetate or acids, salts, enantiomers, analogs, esters, amides, prodrugs, active metabolites, and derivatives thereof;
p. 3α-hydroxyspiro[1αH,5 α H-nortropane-8,1'-pyrrolidinium benzilate or acids, salts, enantiomers, analogs, esters, amides, prodrugs, active metabolites, and derivatives thereof;
q. 4 amino-piperidine containing compounds as disclosed in Diouf et al. (2002) Bioorg. Med. Chem. Lett. 12: 2535-9;
r. pirenzipine or acids, salts, enantiomers, analogs, esters, amides, prodrugs, active metabolites, and derivatives thereof;
s. methoctramine or acids, salts, enantiomers, analogs, esters, amides, prodrugs, active metabolites, and derivatives thereof;
t. 4-diphenylacetoxy-N-methyl piperidine methiodide;
u. tropicamide or acids, salts, enantiomers, analogs, esters, amides, prodrugs, active metabolites, and derivatives thereof;
v. (2R)-N-[1-(6-aminopyridin-2-ylmethyl)piperidin-4-yl]-2-[(1R)-3,3-difluorocyclopentyl]-2-hydroxy-2-phenylacetamide or acids, salts, enantiomers, analogs, esters, amides, prodrugs, active metabolites, and derivatives thereof;
w. PNU-200577 ((R)-N,N-diisopropyl-3-(2-hydroxy-5-hydroxymethylphenyl)-3-phenylpropanamine) or acids, salts, enantiomers, analogs, esters, amides, prodrugs, active metabolites, and derivatives thereof;
x. KRP-197 (4-(2-methylimidazolyl)-2,2-diphenylbutyramide) or acids, salts, enantiomers, analogs, esters, amides, prodrugs, active metabolites, and derivatives thereof;
y. Fesoterodine or acids, salts, enantiomers, analogs, esters, amides, prodrugs, active metabolites, and derivatives thereof; and
z. SPM 7605 (the active metabolite of Fesoterodine), or acids, salts, enantiomers, analogs, esters, amides, prodrugs, active metabolites, and derivatives thereof.

Further compounds that have antimuscarinic activity and that would therefore be useful in the present invention, can be identified or determined by performing muscarinic receptor binding specificity studies as described by Nilvebrant (2002) Pharmacol. Toxicol. 90: 260-7 or cystometry studies as described by Modiri et al. (2002) Urology 59: 963-8.

A further class of compounds which can be used in accordance with the present invention, are adrenergic antagonists or agonists. The term "adrenergic antagonist or agonist" as preferably used herein, is used in its conventional sense to refer to a compound that binds to and antagonizes or agonizes adrenergic receptors. Unless otherwise indicated, the term "adrenergic antagonist or agonist" is intended to include adrenergic antagonists or agonists as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

Adrenergic receptors are cell-surface receptors for two major catecholamine hormones and neurotransmitters: noradrenaline and adrenaline. (Malbon et al. (February 2000) Adrenergic Receptors. In Encyclopedia of Life Sciences. London: Nature Publishing Group, http:/www.els.net). Adrenergic receptors have been implicated in critical physiological processes, including blood pressure control, myocardial and smooth muscle contractility, pulmonary function, metabolism, and central nervous system activity (See, e.g., Malbon et al., Adrenergic Receptors, supra). Two classes of adrenergic receptors have been identified, α and β, that may be further subdivided into three major families (α1, α2, and β), each with at least three subtypes (α1A, B, and, D; α₂A, B, and C; and β1,β2, and β3) based upon their binding characteristics to different agonists and molecular cloning techniques. (See, e.g., Malbon et al., Adrenergic Receptors, supra). It has been shown that β3 adrenergic receptors are expressed in the detrusor muscle, and that the detrusor muscle relaxes with a β3-agonist (Takeda, M. et al. (1999) J. Pharmacol. Exp. Ther. 288: 1367-1373), and in general, β3 adrenergic receptors have been implicated in bladder function (See, e.g., Takeda et al. (2002) Neuourol. Urodyn. 21: 558-65; Takeda et al. (2000) J. Pharmacol. Exp. Ther. 293: 939-45.

Other agents useful in the present invention include any P3 adrenergic agonist agent. Compounds that have been identified as β3 adrenergic agonist agents and that are useful in the present invention include, but are not limited to:
a. TT-138 and phenylethanolamine compounds as disclosed in U.S. Pat. No. 6,069,176, PCT Publication No. WO 97/15549 and available from Mitsubishi Pharma Corp., or acids, salts, esters, amides, prodrugs, active metabolites, and other derivatives thereof;
b. FR-149174 and propanolamine derivatives as disclosed in U.S. Pat. Nos. 6,495,546 and 6,391,915 and available from Fujisawa Pharmaceutical Co., or acids, salts, esters, amides, prodrugs, active metabolites, and other derivatives thereof;
c. KUC-7483, available from Kissei Pharmaceutical Co., or acids, salts, esters, amides, prodrugs, active metabolites, and other derivatives thereof,
d. 4'-hydroxynorephedrine derivatives such as 2-2-chloro-4-(2-((1S,2R)-2-hydroxy-2-(4-hydroxyphenyl)-1-methylethylamino)-ethyl)phenoxy acetic acid as disclosed in Tanaka et al. (2003) J. Med. Chem. 46: 105-12 or acids, salts, esters, amides, prodrugs, active metabolites, and other derivatives thereof;
e. 2-amino-1-phenylethanol compounds, such as BRL35135 ((R*R*)-(.+-.)-[4-[2-[2-(3-chlorophenyl)-2-ydroxyethylamino]propyl]phenoxy]acetic acid methyl ester hydrobromide salt as disclosed in Japanese Patent Publication No. 26744 of 1988 and European Patent Publication No. 23385), and SR58611A ((RS)-N-(7-ethoxycarbonylmethoxy-1,2,3,4-tetrahydronaphth-2-yl)-2-(3-chlorophenyl)-2-hydroxyethanamine hydrochloride as disclosed in Japanese Laid-open Patent Publication No. 66152 of 1989 and European Laid-open Patent Publication No. 255415) or acids, salts, esters, amides, prodrugs, active metabolites, and other derivatives thereof;
f. GS 332 (Sodium (2R)-[3-[3-[2-(3 Chlorophenyl)-2-hydroxyethylamino]cyclohexyl]phenoxy]acetate) as disclosed in Iizuka et al. (1998) J. Smooth Muscle Res. 34: 139-49 or acids, salts, esters, amides, prodrugs, active metabolites, and other derivatives thereof;
g. BRL-37,344 (4-[-[(2-hydroxy-(3-chlorophenyl)ethyl)-amino]propyl]phenoxyacetate) as disclosed in Tsujii et al. (1998) Physiol. Behav. 63: 723-8 and available from GlaxoSmithKline or acids, salts, esters, amides, prodrugs, active metabolites, and other derivatives thereof;
h. BRL-26830A as disclosed in Takahashi et al. (1992) Jpn Circ. J. 56: 936-42 and available from GlaxoSmithKline or acids, salts, esters, amides, prodrugs, active metabolites, and other derivatives thereof;
i. CGP 12177 (4-[3-t-butylamino-2-hydroxypropoxy]benzimidazol-2-one) (a β1/β2 adrenergic antagonist reported to act as an agonist for the β3 adrenergic receptor) as described in Tavernier et al. (1992) J. Pharmacol. Exp. Ther. 263: 1083-90 and available from Ciba-Geigy or acids, salts, esters, amides, prodrugs, active metabolites, and other derivatives thereof;
j. CL 316243 (R,R-5-[2-[[2-(3-chlorophenyl)-2-hydroxyethyl]amino]propyl]-1,3-benzodioxole-2,2-dicarboxylate) as disclosed in Berlan et al. (1994) J. Pharmacol. Exp. Ther. 268: 1444-51 or acids, salts, esters, amides, prodrugs, active metabolites, and other derivatives thereof;
k. Compounds having β3 adrenergic agonist activity as disclosed in US Patent Application 20030018061 or acids, salts, esters, amides, prodrugs, active metabolites, and other derivatives thereof;
1. ICI 215,001 HCl ((S)-4-[2-Hydroxy-3-phenoxypropylaminoethoxy]phenoxyacetic acid hydrochloride) as disclosed in Howe (1993) Drugs Future 18: 529 and available from AstraZeneca/ICI Labs or acids, salts, enantiomers, analogs, esters, amides, prodrugs, active metabolites, and derivatives thereof;
m. ZD 7114 HCl (ICI D7114; (S)-4-[2-Hydroxy-3-phenoxypropylaminoethoxy]-N-(2-methoxyethyl)phenoxyacetamide HCl) as disclosed in Howe (1993) Drugs Future 18: 529 and available from AstraZeneca/ICI Labs or acids, salts, enantiomers, analogs, esters, amides, prodrugs, active metabolites, and derivatives thereof;
n. Pindolol (1-(1H-Indol-4-yloxy)-3-[(1-methylethyl)amino]-2-propanol) as disclosed in Blin et al. (1994) Mol. Pharmacol. 44: 1094 or acids, salts, enantiomers, analogs, esters, amides, prodrugs, active metabolites, and derivatives thereof;
o. (S)-(-)-Pindolol ((S)-1-(1H-indol-4-yloxy)-3-[(1-methylethyl)amino]-2-propanol) as disclosed in Walter et al. (1984) Naunyn-Schmied.Arch.Pharmacol. 327: 159 and Kalkman (1989) Eur. J. Pharmacol. 173: 121 or acids, salts, enantiomers, analogs, esters, amides, prodrugs, active metabolites, and derivatives thereof;
p. SR 59230A HCl (1-(2-Ethylphenoxy)-3-[[(1S)-1,2,3,4-tetrahydro-1-naphthalenyl]amino]-(2S)-2-propanol hydrochloride) as disclosed in Manara et al. (1995) Pharmacol. Comm. 6: 253 and Manara et al. (1996) Br. J. Pharmacol. 117: 435 and available from Sanofi-Midy or acids, salts, enantiomers, analogs, esters, amides, prodrugs, active metabolites, and derivatives thereof;
q. SR 58611 (N[2s)₇carb-ethoxymethoxy-1,2,3,4-tetra-hydronaphth]-(2r)-2-hydroxy-2(3-chlorophenyl) ethamine hydrochloride) as disclosed in Gauthier et al. (1999) J. Pharmacol. Exp. Ther. 290: 687-693 and available from Sanofi Research; and
r. YM178 available from Yamanouchi Pharmaceutical Co. or acids, salts, esters, amides, prodrugs, active metabolites, and other derivatives thereof.

The identification of further compounds that have β3 adrenergic agonist activity and would therefore be useful in the present invention can be determined by performing radioligand binding assays and/or contractility studies as described by Zilberfarb et al. (1997) J. Cell Sci. 110: 801-807; Takeda et al. (1999) J. Pharmacol. Exp. Ther. 288: 1367-1373; and Gauthier et al. (1999) J. Pharmacol. Exp. Ther. 290: 687-693.

A further class of compounds which can be used in accordance with the present invention are "vasopressin agonists". The term "vasopressin agonist" is preferably used in its conventional sense to refer to a compound that binds to and activates vasopressin receptors. Unless otherwise indicated, the term "vasopressin agonist" is intended to include vasopressin receptor agonists, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

A further class of compounds which can be used in accordance with the present invention are neurokinin antagonists. The term "neurokinin antagonist" is preferably used in its conventional sense to refer to a compound that binds to and antagonizes neurokinin receptors. Unless otherwise indicated, the term "neurokinin antagonist" is intended to include neurokinin receptor antagonist agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

Tachykinins (TKs) are a family of structurally related peptides that include substance P, neurokinin A (NKA) and neurokinin B (NKB). Neurons are the major source of TKs in the periphery. An important general effect of TKs is neuronal stimulation, but other effects include endothelium-dependent vasodilation, plasma protein extravasation, mast cell recruitment and degranulation and stimulation of inflammatory cells (See Maggi, C. A. (1991) Gen. Pharmacol., 22: 1-24). In general, tachykinin receptors have been implicated in bladder function (See, e.g., Kamo et al. (2000) Eur. J. Pharmacol. 401: 235-40 and Omhura et al. (1997) Urol. Int. 59: 221-5).

Substance P activates the neurokinin receptor subtype referred to as NK₁. Substance P is an undecapeptide that is present in sensory nerve terminals. Substance P is known to have multiple actions that produce inflammation and pain in the periphery after C-fibre activation, including vasodilation, plasma extravasation and degranulation of mast cells (Levine, J. D. et. al. (1993) J. Neurosci. 13: 2273).

Neurokinin A is a peptide which is colocalized in sensory neurons with substance P and which also promotes inflammation and pain. Neurokinin A activates the specific neurokinin receptor referred to as NK₂ (Edmonds-Alt, S., et. al. (1992) Life Sci. 50: PL101). In the urinary tract, TKs are powerful spasmogens acting through only the NK₂ receptor in the human bladder, as well as the human urethra and ureter (Maggi, C. A. (1991) Gen. Pharmacol., 22: 1-24).

Insofar, agents useful in the present invention thus include any neurokinin receptor antagonist agent. Suitable neurokinin receptor antagonists for use in the present invention that act preferably on the NK₁ receptor include, but are not limited to: 1-imino-2-(2-methoxy-phenyl)-ethyl)-7,7-diphenyl-4-perhydroisoindolone(3aR,7aR) ("RP 67580"); 2S,3S-cis-3-(2-methoxybenzyla- mino)-2-benzhydrylquinuclidine ("CP 96,345"); and (aR,9R)-7-[3,5-bis(trifl- uoromethyl)benzyl]-8,9,10,11-tetrahydro-9-methyl-5-(4-methylphenyl)-7H-[1,4]diazocino[2,1-g][1,7]naphthyridine-6,13-dione) ("TAK-637"). Suitable neurokinin receptor antagonists for use in the present invention that preferably act on the NK₂ receptor include but are not limited to: (S)-N-methyl-N-4-(4-acetylamino-4-phenylpiperidino)-2-(3,4-dichloropheny- 1)butylbenzamide ("SR 48968"); Met-Asp-Trp-Phe-Dap-Leu ("MEN 10,627"); and cyc(Gln-Trp-Phe-Gly-Leu-Met) ("L 659,877"). Suitable neurokinin receptor antagonists for use in the present invention also include acids, salts, esters, amides, prodrugs, active metabolites, and other derivatives of any of the agents mentioned above. The identification of further compounds that have neurokinin receptor antagonist activity and would therefore be useful in the present invention can be determined by performing binding assay studies as described in Hopkins et al. (1991) Biochem. Biophys. Res. Comm. 180: 1110-1117; and Aharony et al. (1994) Mol. Pharmacol. 45: 9-19.

A further class of compounds which can be used in accordance with the present invention are potassium channel activators. The term "potassium channel activator" is preferably used in its conventional sense to refer to a compound that binds to and agonizes potassium channels. Unless otherwise indicated, the term "potassium channel activator" is intended to include potassium channel activator agents, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

A further class of compounds which can be used in accordance with the present invention are NO donors. The term "NO donor" is preferably used in its conventional sense to refer to a compound that releases free nitric oxide when administered to a patient. Unless otherwise indicated, the term "NO donor" is intended to include nitric oxide donor agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

Nitric oxide donors may be included in the present invention particularly for their anti-spasm activity. Nitric oxide (NO) plays a critical role as a molecular mediator of many physiological processes, including vasodilation and regulation of normal vascular tone. The action of NO is implicated in intrinsic local vasodilation mechanisms. NO is the smallest biologically active molecule known and is the mediator of an extraordinary range of physiological processes (Nathan (1994) Cell 78: 915-918; Thomas (1997) Neurosurg. Focus 3: Article 3). NO is also a known physiologic antagonist of endothelin-1, which is the most potent known mammalian vasoconstrictor, having at least ten times the vasoconstrictor potency of angiotensin II (Yanagisawa et al. (1988) Nature 332: 411-415; Kasuya et al. (1993) J. Neurosurg. 79: 892-898; Kobayashi et al., (1991) Neurosurgery 28: 673-679). The biological half-life of NO is extremely short (Morris et al. (1994) Am. J. Physiol. 266: E829-E839; Nathan (1994) Cell 78: 915-918). NO accounts entirely for the biological effects of endothelium-derived relaxing factor (EDRF) and is an extremely potent vasodilator that is believed to work through the action of cGMP-dependent protein kinases to effect vasodilation (Henry et al. (1993) FASEB J. 7: 1124-1134; Nathan (1992) FASEB J. 6: 3051-3064; Palmer et al., (1987) Nature 327: 524-526; Snyder et al. (1992) Scientific American 266: 68-77).

Within endothelial cells, an enzyme known as NO synthase (NOS) catalyzes the conversion of L-arginine to NO which acts as a diffusible second messenger and mediates responses in adjacent smooth muscle cells. NO is continuously formed and released by the vascular endothelium under basal conditions which inhibits contractions and controls basal coronary tone and is produced in the endothelium in response to various agonists (such as acetylcholine) and other endothelium dependent vasodilators. Thus, regulation of NOS activity and the resultant levels of NO are key molecular targets controlling vascular tone (Muramatsu et. al. (1994) Coron. Artery Dis. 5: 815-820).

Insofar, agents useful in the present invention thus include any nitric oxide donor agent. Suitable nitric oxide donors for the practice of the present invention include but are not limited to:
a. Nitroglycerine or acids, salts, enantiomers, analogs, esters, amides, prodrugs, active metabolites, and derivatives thereof;
b. Sodium nitroprusside or acids, salts, enantiomers, analogs, esters, amides, prodrugs, active metabolites, and derivatives thereof;
c. FK 409 (NOR-3) or acids, salts, enantiomers, analogs, esters, amides, prodrugs, active metabolites, and derivatives thereof;
d. FR 144420 (NOR-4) or acids, salts, enantiomers, analogs, esters, amides, prodrugs, active metabolites, and derivatives thereof;
e. 3-morpholinosydnonimine or acids, salts, enantiomers, analogs, esters, amides, prodrugs, active metabolites, and derivatives thereof;
f. Linsidomine chlorohydrate ("SIN-1") or acids, salts, enantiomers, analogs, esters, amides, prodrugs, active metabolites, and derivatives thereof;
g. S-nitroso-N-acetylpenicillamine ("SNAP") or acids, salts, enantiomers, analogs, esters, amides, prodrugs, active metabolites, and derivatives thereof;
h. AZD3582 (CINOD lead compound, available from NicOx S.A.) or acids, salts, enantiomers, analogs, esters, amides, prodrugs, active metabolites, and derivatives thereof;
i. NCX 4016 (available from NicOx S.A.) or acids, salts, enantiomers, analogs, esters, amides, prodrugs, active metabolites, and derivatives thereof;
j. NCX 701 (available from NicOx S.A.) or acids, salts, enantiomers, analogs, esters, amides, prodrugs, active metabolites, and derivatives thereof;
k. NCX 1022 (available from NicOx S.A.) or acids, salts, enantiomers, analogs, esters, amides, prodrugs, active metabolites, and derivatives thereof;
1. HCT 1026 (available from NicOx S.A.) or acids, salts, enantiomers, analogs, esters, amides, prodrugs, active metabolites, and derivatives thereof;
m. NCX 1015 (available from NicOx S.A.) or acids, salts, enantiomers, analogs, esters, amides, prodrugs, active metabolites, and derivatives thereof;
n. NCX 950 (available from NicOx S.A.) or acids, salts, enantiomers, analogs, esters, amides, prodrugs, active metabolites, and derivatives thereof;
o. NCX 1000 (available from NicOx S.A.) or acids, salts, enantiomers, analogs, esters, amides, prodrugs, active metabolites, and derivatives thereof;
p. NCX 1020 (available from NicOx S.A.) or acids, salts, enantiomers, analogs, esters, amides, prodrugs, active metabolites, and derivatives thereof;
q. AZD 4717 (available from NicOx S.A.) or acids, salts, enantiomers, analogs, esters, amides, prodrugs, active metabolites, and derivatives thereof;
r. NCX 1510/NCX 1512 (available from NicOx S.A.) or acids, salts, enantiomers, analogs, esters, amides, prodrugs, active metabolites, and derivatives thereof;
s. NCX 2216 (available from NicOx S.A.) or acids, salts, enantiomers, analogs, esters, amides, prodrugs, active metabolites, and derivatives thereof;
t. NCX 4040 (available from NicOx S.A.) or acids, salts, enantiomers, analogs, esters, amides, prodrugs, active metabolites, and derivatives thereof;
u. Nitric oxide donors as disclosed in U.S. Pat. No. 5,155,137 or acids, salts, enantiomers, analogs, esters, amides, prodrugs, active metabolites, and derivatives thereof;
v. Nitric oxide donors as disclosed in U.S. Pat. No. 5,366,997 or acids, salts, enantiomers, analogs, esters, amides, prodrugs, active metabolites, and derivatives thereof;
w. Nitric oxide donors as disclosed in U.S. Pat. No. 5,405,919 or acids, salts, enantiomers, analogs, esters, amides, prodrugs, active metabolites, and derivatives thereof;
x. Nitric oxide donors as disclosed in U.S. Pat. No. 5,650,442 or acids, salts, enantiomers, analogs, esters, amides, prodrugs, active metabolites, and derivatives thereof;
y. Nitric oxide donors as disclosed in U.S. Pat. No. 5,700,830 or acids, salts, enantiomers, analogs, esters, amides, prodrugs, active metabolites, and derivatives thereof;
z. Nitric oxide donors as disclosed in U.S. Pat. No. 5,632,981 or acids, salts, enantiomers, analogs, esters, amides, prodrugs, active metabolites, and derivatives thereof;

aa. Nitric oxide donors as disclosed in U.S. Pat. No. 6,290,981 or acids, salts, enantiomers, analogs, esters, amides, prodrugs, active metabolites, and derivatives thereof;
bb. Nitric oxide donors as disclosed in U.S. Pat. No. 5,691,423 or acids, salts, enantiomers, analogs, esters, amides, prodrugs, active metabolites, and derivatives thereof;
cc. Nitric oxide donors as disclosed in U.S. Pat. No. 5,721,365 or acids, salts, enantiomers, analogs, esters, amides, prodrugs, active metabolites, and derivatives thereof;
dd. Nitric oxide donors as disclosed in U.S. Pat. No. 5,714,511 or acids, salts, enantiomers, analogs, esters, amides, prodrugs, active metabolites, and derivatives thereof;
ee. Nitric oxide donors as disclosed in U.S. Pat. No. 6,511,911 or acids, salts, enantiomers, analogs, esters, amides, prodrugs, active metabolites, and derivatives thereof; and
ff. Nitric oxide donors as disclosed in U.S. Pat. No. 5,814,666.

The identification of further compounds that have nitric oxide donor activity and that would therefore be useful in the present invention can be determined by release profile and/or induced vasospasm studies as described in U.S. Pat. Nos. 6,451,337 and 6,358,536, as well as Moon (2002) IBJU Int. 89: 942-9 and Fathian-Sabet et al. (2001) J. Urol. 165: 1724-9.

A further class of compounds which can be used in accordance with the present invention are calcium modulators. The term "calcium modulator" or "Ca²⁺ modulator" as preferably used herein refers to an agent that is capable of interacting with a calcium channel, including a binding event, including subtypes of the calcium channel subunits as disclosed in Klugbauer et al. (1999) J. Neurosci. 19: 684-691, to produce a physiological effect, such as opening, closing, blocking, up-regulating functional expression, down-regulating functional expression, or desensitization, of the channel. Unless otherwise indicated, the term "calcium modulator" is intended to include, but is not limited to, GABA analogs (e.g. gabapentin and pregabalin), fused bicyclic or tricyclic amino acid analogs of gabapentin, amino acid compounds, and other compounds that interact with the calcium channels as further disclosed herein, as well as acids, salts, esters, amides, prodrugs, active metabolites, and other derivatives thereof.

Further, it is understood that any salts, esters, amides, prodrugs, active metabolites or other derivatives are pharmaceutically acceptable as well as pharmacologically active.

Gamma-aminobutyric acid (GABA) analogs are compounds that are derived from or based on GABA. GABA analogs are either readily available or readily synthesized using methodologies known to those of skill in the art. Exemplary GABA analogs include gabapentin and pregabalin.

Gabapentin (Neurontin, or 1-(aminomethyl) cyclohexaneacetic acid) is an anticonvulsant drug with a high binding affinity for some calcium channel subunits, and is represented by the following structure:

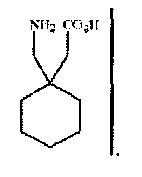

Gabapentin is one of a series of compounds of the following formula: 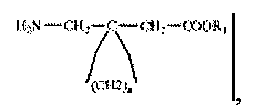 in which R₁ is hydrogen or a lower alkyl radical and n is 4, 5, or 6. Although gabapentin was originally developed as a GABA-mimetic compound to treat spasticity, gabapentin has no direct GABAergic action and does not block GABA uptake or metabolism. (For review, see Rose et al. (2002) Analgesia 57:451-462). Gabapentin has been found, however, to be an effective treatment for the prevention of partial seizures in patients who are refractory to other anticonvulsant agents (Chadwick (1991) Gabapentin, In Pedley T A, Meldrum B S (eds.), Recent Advances in Epilepsy, Churchill Livingstone, New York, pp. 211-222). Gabapentin and the related drug pregabalin may interact with the α₂δ subunit of calcium channels (Gee et al. (1996) J. Biol. Chem. 271: 5768-5776).

In addition to its known anticonvulsant effects, gabapentin has been shown to block the tonic phase of nociception induced by formalin and carrageenan, and exerts an inhibitory effect in neuropathic pain models of mechanical hyperalgesia and mechanical/thermal allodynia (Rose et al. (2002) Analgesia 57: 451-462). Double-blind, placebo-controlled trials have indicated that gabapentin is an effective treatment for painful symptoms associated with diabetic peripheral neuropathy, post-herpetic neuralgia, and neuropathic pain (see, e.g., Backonja et al. (1998) JAMA 280:1831-1836; Mellegers et al. (2001) Clin. J. Pain 17:284-95).

Pregabalin, (S)-(3-aminomethyl)-5-methylhexanoic acid or (S)-isobutyl GABA, is another GABA analog whose use as an anticonvulsant has been explored (Bryans et al. (1998) J. Med. Chem. 41:1838-1845).

Exemplary GABA analogs and fused bicyclic or tricyclic amino acid analogs of gabapentin that are useful in the present invention include:
1. Gabapentin or salts, enantiomers, analogs, esters, amides, prodrugs, active metabolites, or derivatives thereof;
2. Pregabalin or salts, enantiomers, analogs, esters, amides, prodrugs, active metabolites, or derivatives thereof;
3. GABA analogs according to the following structure as described in U.S. Pat. No. 4,024,175, or salts, enantiomers, analogs, esters, amides, prodrugs, active metabolites, or derivatives thereof, 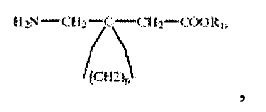 wherein R₁ is hydrogen or a lower alkyl radical and n is 4, 5, or 6;
4. GABA analogs according to the following structure as described in U.S. Pat. No. 5,563,175, or salts, enantiomers, analogs, esters, amides, prodrugs, active metabolites, or derivatives thereof, 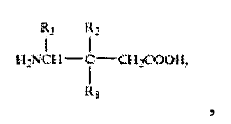 wherein R₁ is a straight or branched alkyl group having from 1 to 6 carbon atoms, phenyl, or cycloalkyl having from 3 to 6 carbon atoms; R₂ is hydrogen or methyl; and R₃ is hydrogen, methyl or carboxyl;
5. Substituted amino acids according to the following structures as described in U.S. Pat. No. 6,316,638, or salts, enantiomers, analogs, esters, amides, prodrugs, active metabolites, or derivatives thereof, 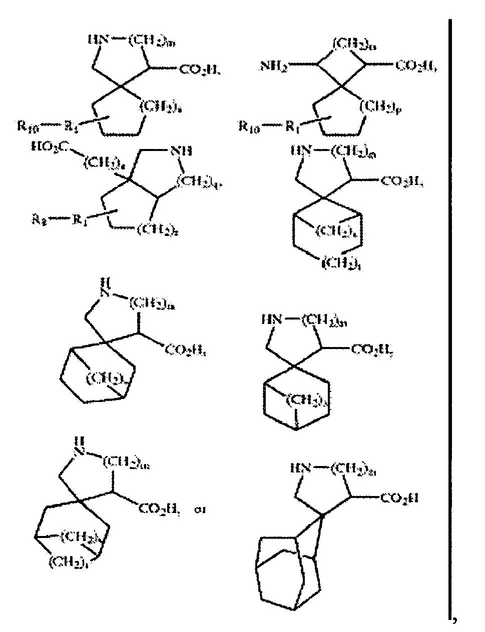 wherein R₁ to R₁₀ are each independently selected from hydrogen or a straight or branched alkyl of from 1 to 6 carbons, benzyl, or phenyl; m is an integer of from 0 to 3; n is an integer from 1 to 2; o is an integer from 0 to 3; p is an integer from 1 to 2; q is an integer from 0 to 2; r is an integer from 1 to 2; s is an integer from 1 to 3; t is an integer from 0 to 2; and u is an integer from 0 to 1;
6. GABA analogs as disclosed in PCT Publication No. WO 93/23383 or salts, enantiomers, analogs, esters, amides, prodrugs, active metabolites, or derivatives thereof;
7. GABA analogs as disclosed in Bryans et al. (1998) J. Med. Chem. 41:1838-1845 or salts, enantiomers, analogs, esters, amides, prodrugs, active metabolites, or derivatives thereof;
8. GABA analogs as disclosed in Bryans et al. (1999) Med. Res. Rev. 19:149-177 or salts, enantiomers, analogs, esters, amides, prodrugs, active metabolites, or derivatives thereof;
9. Amino acid compounds according to the following structure as described in U.S. Application No. 20020111338, or salts, enantiomers, analogs, esters, amides, prodrugs, active metabolites, or derivatives thereof; 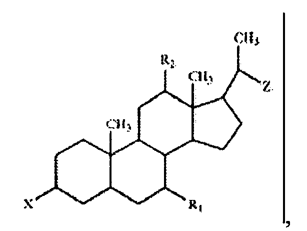 wherein R₁ and R₂ are independently hydrogen or hydroxy; X is selected from the group consisting of hydroxy and Q₂-G- where:
   G is ―O―, ―C(O)O― or ―NH―;
   Q^{X} is a group derived from a linear oligopeptide comprising a first moiety D and further comprising from 1 to 3 amino acids, and wherein said group is cleavable from the amino acid compound under physiological conditions;
   D is a GABA analog moiety;
   Z is selected from the group consisting of:
      (i) a substituted alkyl group containing a moiety which is negatively charged at physiological pH, which moiety is selected from the group consisting of ―COOH, ―SO₃H, ―SO₂H, ― P(O)(OR¹⁶)(OH), ―OP(O)(OR¹⁶)(OH), ―OSO₃H and the like, and where R¹⁶ is selected from the group consisting of alkyl, substituted alkyl, aryl and substituted aryl; and
      (ii) a group of the formula -M-Q^{x'}, wherein M is selected from the group consisting of ― CH₂OC(O) ― and ―CH₂CH₂C(O) ―, and wherein Q^{x'} is a group derived from a linear oligopeptide comprising a first moiety D' and further comprising from 1 to 3 amino acids, and wherein said group is cleavable under physiological conditions; D' is a GABA analog moiety; or a pharmaceutically acceptable salt thereof; provided that when X is hydroxy, then Z is a group of formula -M-Q^{x'};
10. Cyclic amino acid compounds as disclosed in PCT Publication No. WO 99/08670 or salts, enantiomers, analogs, esters, amides, prodrugs, active metabolites, or derivatives thereof;
11. Cyclic amino acids according to the following structures as disclosed in PCT Publication No. WO99/21824, or salts, enantiomers, analogs, esters, amides, prodrugs, active metabolites, or derivatives thereof, 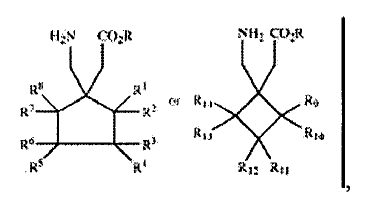 wherein R is hydrogen or a lower alkyl; R₁ to R₁₄ are each independently selected from hydrogen, straight or branched alkyl of from 1 to 6 carbons, phenyl, benzyl, fluorine, chlorine, bromine, hydroxy, hydroxymethyl, amino, aminomethyl, trifluoromethyl, ―CO₂H, ― CO₂R₁₅, ―CH₂CO₂H, ―CHCO₂R₁₅, ―OR₁₅ wherein R₁₅ is a straight or branched alkyl of from 1 to 6 carbons, phenyl, or benzyl, and R₁ to R₈ are not simultaneously hydrogen;
12. Bicyclic amino acids according to the following structures as disclosed in published U.S. Patent Application Serial No. 60/160,725, including those disclosed as having high activity as measured in a radioligand binding assay using [3H]gabapentin and the α₂δ subunit derived from porcine brain tissue, or acids, salts, enantiomers, analogs, esters, amides, prodrugs, active metabolites, and derivatives thereof, 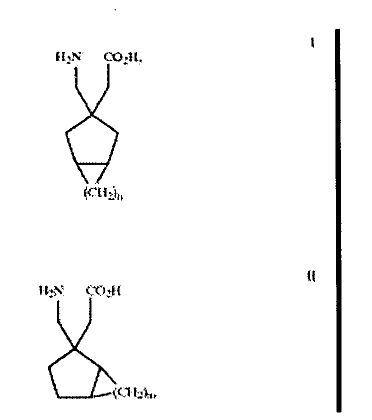 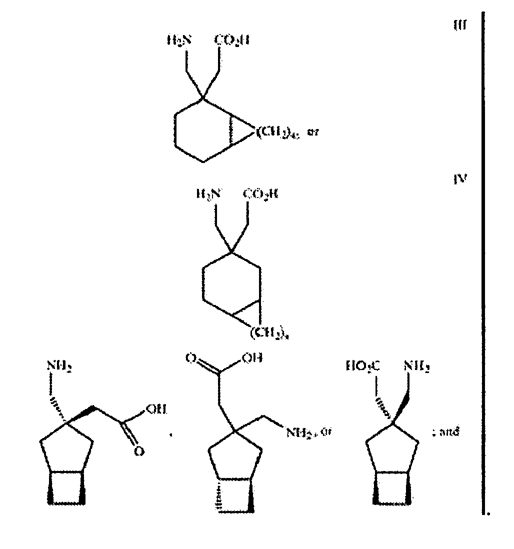 whereby n is any integer from 2 to 6.
13. Bicyclic amino acid analogs according to the following structures as disclosed in UK Patent Application GB 2 374 595 and acids, salts, enantiomers, analogs, esters, amides, prodrugs, active metabolites, and derivatives thereof. 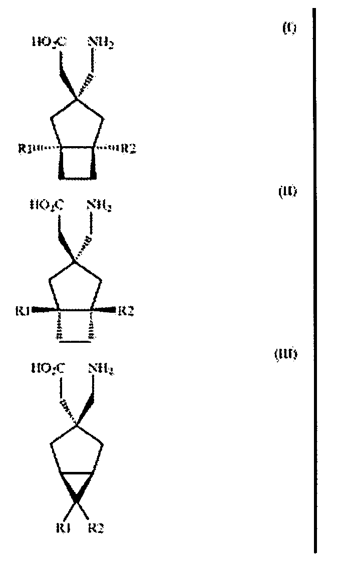 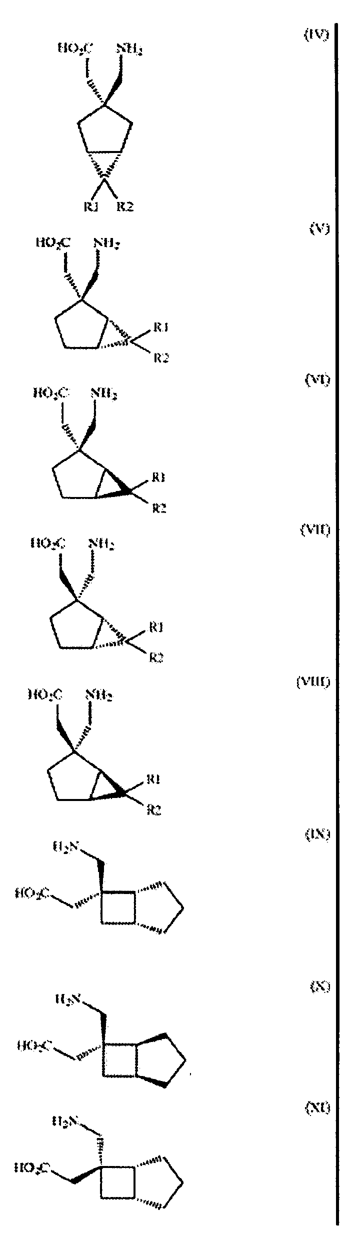 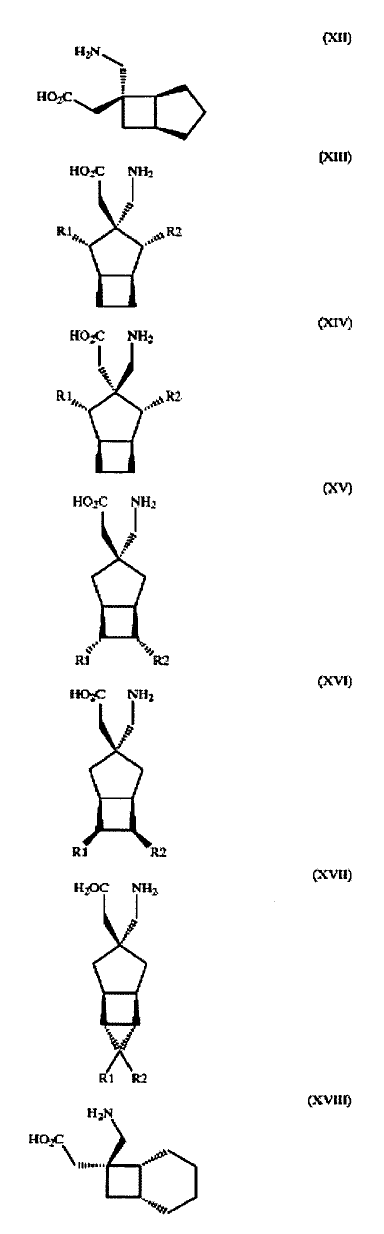 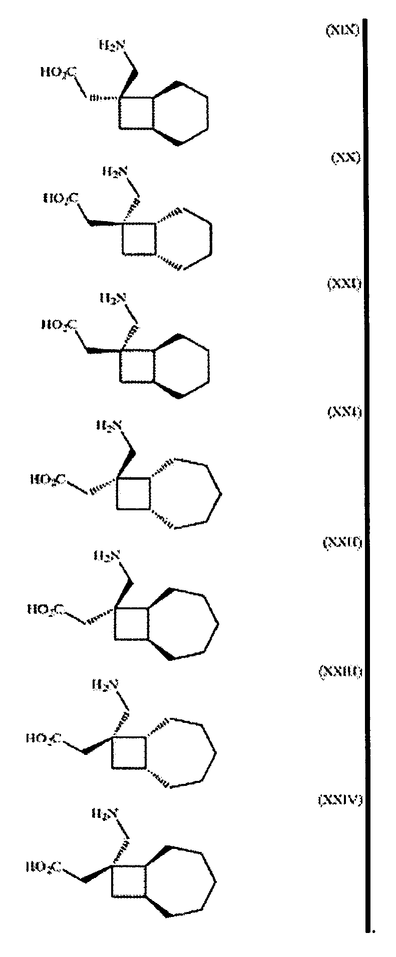 whereby R₁ and R₂ are each and independently selected from hydrogen, straight or branched alkyl of from 1 to 6 carbons, phenyl, benzyl, fluorine, chlorine, bromine, hydroxy, hydroxymethyl, amino, aminomethyl, trifluoromethyl, ―CO₂H, ―CO₂R₁₅, ―CH₂CO₂H, ― CHCO₂R₁₅, ―OR₁₅ wherein R₁₅ is a straight or branched alkyl of from 1 to 6 carbons, phenyl, or benzyl, and R₁ to R₈ are not simultaneously hydrogen;

Other agents useful in the present invention include any compound that binds to the α₂δ subunit of a calcium channel. GABA analogs which display binding affinity to the α₂δ subunit of calcium channels and that are therefore useful in the present invention include, without limitation, cis-(1S,3R)-(1-(aminomethyl)-3-methylcyclohexane)acetic acid, cis-(1R,3S)-(1-(aminomethyl)-3-methylcyclohexa- ne)acetic acid, 1α,3α,5α-(1-aminomethyl)-(3,5-dimethylcyclohexane)acetic acid, (9-(aminomethyl)bicyclo[3.3.1]non-9-yl)acetic acid, and (7-(aminomethyl)bicyclo[2.2.1]hept-7-yl)acetic acid (Bryans et al. (1998) J. Med. Chem. 41:1838-1845; Bryaris et al. (1999) Med. Res. Rev. 19:149-177). Other compounds that have been identified as modulators of calcium channels include, but are not limited to those described in U.S. Pat. No. 6,316,638, U.S. Pat. No. 6,492,375, U.S. Pat. No. 6,294,533, U.S. Pat. No. 6,011,035, U.S. Pat. No. 6,387,897, U.S. Pat. No. 6,310,059, U.S. Pat. No. 6,294,533, U.S. Pat. No. 6,267,945, PCT Publication No. WO01/49670, PCT Publication No. WO01/46166, and PCT Publication No. WO01/45709. The identification of which of these compounds have a binding affinity for the α₂δ subunit of calcium channels can be determined by performing α₂δ binding affinity studies as described by Gee et al. (Gee et al. (1996) J. Biol. Chem. 271:5768-5776). The identification of still further compounds, including other GABA analogs, that exhibit binding affinity for the α₂δ subunit of calcium channels can also be determined by performing α₂δ binding affinity studies as described by Gee et al. (Gee et al. (1996) J. Biol. Chem. 271:5768-5776).

Furthermore, compositions and formulations encompassing GABA analogs and cyclic amino acid analogs of gabapentin and that would be useful in the present invention include compositions disclosed in PCT Publication No. WO 99/08670, U.S. Pat. No. 6,342,529, controlled release formulations as disclosed in U.S. Application No. 20020119197 and U.S. Pat. No. 5,955,103, and sustained release compounds and formulations as disclosed in PCT Publication No. WO 02/28411, PCT Publication No. WO 02/28881, PCT Publication No. WO 02/28883, PCT Publication No. WO 02/32376, PCT Publication No. WO 02/42414, U.S. Application No. 20020107208, U.S. Application No. 20020151529, and U.S. Application No. 20020098999.

A further class of compounds which can be used in accordance with the present invention are spasmolyticy. The term "spasmolytic" (also known as "antispasmodic") is preferably used in its conventional sense to refer to a compound that relieves or prevents muscle spasms, especially of smooth muscle. Unless otherwise indicated, the term "spasmolytic" is intended to include spasmolytic agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

Spasmolytics are compounds that relieve or prevent muscle spasms, especially of smooth muscle. In general, spasmolytics have been implicated as having efficacy in the treatment of bladder disorders (See e.g., Takeda et al. (2000) J. Pharmacol. Exp. Ther. 293: 939-45).

Other agents useful in the present invention include any spasmolytic agent. Compounds that have been identified as spasmolytic agents and are useful in the present invention include, but are not limited to:
a. α-α-diphenylacetic acid-4-(N-methyl-piperidyl) esters as disclosed in U.S. Pat. No. 5,897,875 or acids, salts, enantiomers, analogs, esters, amides, prodrugs, active metabolites, and derivatives thereof;
b. Human and porcine spasmolytic polypeptides in glycosylated form and variants thereof as disclosed in U.S. Pat. No. 5,783,416 or acids, salts, enantiomers, analogs, esters, amides, prodrugs, active metabolites, and derivatives thereof;
c. Dioxazocine derivatives as disclosed in U.S. Pat. No. 4,965,259 or acids, salts, enantiomers, analogs, esters, amides, prodrugs, active metabolites, and derivatives thereof;
d. Quaternary 6,11-dihydro-dibenzo-[b,e]-thiepine-11-N-alkylnorscopine ethers as disclosed in U.S. Pat. No. 4,608,377 or acids, salts, enantiomers, analogs, esters, amides, prodrugs, active metabolites, and derivatives thereof;
e. Quaternary salts of dibenzo[1,4]diazepinones, pyrido-[1,4]benzodiazepinones, pyrido[1,5]benzodiazepinones as disclosed in U.S. Pat. No. 4,594,190 or acids, salts, enantiomers, analogs, esters, amides, prodrugs, active metabolites, and derivatives thereof;
f. Endo-8,8-dialkyl-8-azoniabicyclo (3.2.1) octane-6,7-exo-epoxy-3-alkyl-carboxylate salts as disclosed in U.S. Pat. No. 4,558,054 or acids, salts, enantiomers, analogs, esters, amides, prodrugs, active metabolites, and derivatives thereof;
g. Pancreatic spasmolytic polypeptides as disclosed in U.S. Pat. No. 4,370,317 or acids, salts, enantiomers, analogs, esters, amides, prodrugs, active metabolites, and derivatives thereof;
h. Triazinones as disclosed in U.S. Pat. No. 4,203,983 or acids, salts, enantiomers, analogs, esters, amides, prodrugs, active metabolites, and derivatives thereof;
i. 2-(4-Biphenylyl)-N-(2-diethylaminoalkyl)propionamide as disclosed in U.S. Pat. No. 4,185,124 or acids, salts, enantiomers, analogs, esters, amides, prodrugs, active metabolites, and derivatives thereof;
j. Piperazino-pyrimidines as disclosed in U.S. Pat. No. 4,166,852 or acids, salts, enantiomers, analogs, esters, amides, prodrugs, active metabolites, and derivatives thereof;
k. Aralkylamino carboxylic acids as disclosed in U.S. Pat. No. 4,163,060 or acids, salts, enantiomers, analogs, esters, amides, prodrugs, active metabolites, and derivatives thereof;
1. Aralkylamino sulfones as disclosed in U.S. Pat. No. 4,034,103 or acids, salts, enantiomers, analogs, esters, amides, prodrugs, active metabolites, and derivatives thereof;
m. Smooth muscle spasmolytic agents as disclosed in U.S. Pat. No. 6,207,852 or acids, salts, enantiomers, analogs, esters, amides, prodrugs, active metabolites, and derivatives thereof; and
n. Papaverine or acids, salts, enantiomers, analogs, esters, amides, prodrugs, active metabolites, and derivatives thereof.

The identification of further compounds that have spasmolytic activity and would therefore be useful in the present invention can be determined by performing bladder strip contractility studies as described in U.S. Pat. No. 6,207,852; Noronha-Blob et al. (1991) J. Pharmacol. Exp. Ther.256: 562-567; and/or Kachur et al. (1988) J. Pharmacol. Exp. Ther. 247: 867-872.

A further class of compounds which can be used in accordance with the present invention are smooth muscle modulators. The term "smooth muscle modulator" as preferably used herein refers to any compound that inhibits or blocks the contraction of smooth muscles, including but not limited to antimuscarinics, β3 adrenergic agonists, spasmolytics, neurokinin receptor antagonists, bradykinin receptor antagonists, and nitric oxide donors. Smooth muscle modulators can be "direct" (also known as "musculotropic") or "indirect" (also known as "neurotropic"). "Direct smooth muscle modulators" are smooth muscle modulators that act by inhibiting or blocking contractile mechanisms within smooth muscle, including but not limited to modification of the interaction between actin and myosin. "Indirect smooth muscle modulators" are smooth muscle modulators that act by inhibiting or blocking neurotransmission that results in the contraction of smooth muscle, including but not limited to blockade of presynaptic facilitation of acetylcholine release at the axon terminal of motor neurons terminating in smooth muscle.

A further class of compounds which can be used in accordance with the present invention are 5HT re-uptake inhibitors. The term "5HT re-uptake inhibitor" as preferably used herein refers to any compound which can inhibit 5-HT (5-hydroxytryptamine, Serotonin) receptor function, preferably by targeting 5-HT_{2C} and 5-HT₃ receptor subtypes. Unless otherwise indicated, the term "5HT re-uptake inhibitors" is intended to include 5HT re-uptake inhibitor agents, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

A further class of compounds which can be used in accordance with the present invention are purinergic receptor antagonists. The term "purinergic receptor antagonist" is preferably used in its conventional sense to refer to a compound that binds to and antagonizes purinergic receptors. Unless otherwise indicated, the term "purinergic receptor antagonist" is intended to include purinergic receptor antagonist agents, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

A further class of compounds which can be used in accordance with the present invention are PDE inhibitors. The term "PDE inhibitor" is preferably used in its conventional sense to refer to a compound that binds to and antagonizes phosphodiesterases (PDEs). Unless otherwise indicated, the term "PDE inhibitor" is intended to include PDE inhibitor agents, as well as acids, salts, esters, amides, prodrugs, active metabolites, and other derivatives thereof.

Further, it is understood that any salts, esters, amides, prodrugs, active metabolites or other derivatives are pharmaceutically acceptable as well as pharmacologically active.

A further class of compounds which can be used in accordance with the present invention are VR1 modulators. The term "VR1 modulator" is preferably used herein in its conventional sense to refer to a compound that binds to and modulates activity of vanilloid receptor 1 (VR1, transient receptor potential vanniloid-1, TRPV-1). Examples for VR1 modulators are VR1 agonists (capsaicin and resiniferatoxin [RTX], Lopez-Rodriguez et al., Mini Rev Med Chem. 2003 Nov;3(7):729-48). After an initial nerve excitation phase, VR1 agonists are able to induce a desensitized state in nerves (e.g. sensory C-fibres) in which nerve endings are unresponsive to stimuli and neurotransmitters are not released, thereby leading to long-term analgesia. VR1 antagonists (e.g. Capsazepine (Valenzano & Sun, Curr Med Chem. 2004 Dec;11(24):3185-202)) block VR1 receptor activation. Unless otherwise indicated, the term "VR1 modulator" is intended to include VR1 modulator agents, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

A further class of compounds which can be used in accordance with the present invention are analgesics. The term "analgesic" as preferably used herein refers to any compound or drug, whose primary purpose is pain relief. Examples for primary classes of analgesics but not limited to are nonsteroidal anti-inflammatory drugs (NSAIDs) including COX-2 inhibitors, opioids, antidepressants and anticonvulsants. Unless otherwise indicated, the term "analgesic" is intended to include analgesic agents, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

As used herein, each of the following terms, used alone or in conjunction with other terms, are preferably defined as follows (except where noted to the contrary):

The term "alkyl" refers to a saturated aliphatic radical containing from one to fourteen carbon atoms or a mono- or polyunsaturated aliphatic hydrocarbon radical containing from two to twelve carbon atoms, containing at least one double and triple bound, respectively. "Alkyl" refers to both branched and unbranched alkyl groups. Preferred alkyl groups are straight chain alkyl groups containing from one to eight carbon atoms. More preferred alkyl groups are straight chain alkyl groups containing from one to six carbon atoms and branched alkyl groups containing from three to six carbon atoms. It should be understood that any combination term using an "alk" or "alkyl" prefix refers to analogs according to the above definition of "alkyl". For example, terms such as "alkoxy", "alkylthio" refer to alkyl group linked to a second group via an oxygen or sulfur atom. "Alkanoyl" refers to an alkyl group linked to a carbonyl group (C=O). "Substituted alkyl" refers to alkyl groups straight or branched further bearing one or more substituents. One substituent also means monosubstituted and more substitutents mean poly-substituted. It should be understood that any combination term using a "substituted alkyl" prefix refers to analogs according to the above definition of "substituted alkyl". For example, a term such as "substituted alkylaryl" refers to substituted alkyl group linked to an aryl group.

The term "lower alkyl" as used herein is preferably any alkyl as disclosed herein, whereby the alkyl comprises one to six, preferably one to five, and more preferably one or four C-atoms.

The term "cycloalkyl" refers to the cyclic analog of an alkyl group, as defined above, optionally unsaturated and/or substituted. Preferred cycloalkyl groups are saturated cycloalkyl groups, more particularly those containing from three to eight carbon atoms, and even more preferably three to six carbon atoms. "Substituted cycloalkyl" refers to cycloalkyl groups further bearing one or more substituents. "Mono―unsaturated cycloalkyl" refers to cycloalkyl containing one double bond or one triple bond. "Poly―unsaturated cycloalkyl" refers to cycloalkyl containing at least two double bonds or two triple bonds or a combination of at least one double bond and one triple bond.

The term "alkenyl" refers to an unsaturated hydrocarbon group containing at least one carbon-carbon double bond, including straight-chain, branched-chain, and cyclic groups. Preferred alkenyl groups have one to twelve carbons. More preferred alkenyl groups have one to six carbons. "Substituted alkenyl" refers to alkenyl groups further bearing one or more substitutents.

The term "cycloalkenyl" refers to the cyclic analog of an alkenyl group, as defined above, optionally substituted. Preferred cycloalkenyl groups are containing from four to eight carbon atoms. "Substituted cycloalkenyl" refers to cycloalkenyl groups further bearing one or more substituents. "Mono―unsaturated cycloalkenyl" refers to cycloalkenyl containing one double bond. "Poly―unsaturated cycloalkenyl" refers to cycloalkenyl containing at least two double bonds.

The term "alkynyl" refers to an unsaturated hydrocarbon group containing at least one carbon-carbon triple bond, including straight-chain, branched-chain, and cyclic groups. Preferred alkynyl groups have one to twelve carbons. More preferred alkynyl groups have one to six carbons. "Substituted alkynyl" refers to alkynyl groups further bearing one or more substitutents.

The term "aryl" refers to aromatic groups having in the range of 6 to 14 carbon atoms and "substituted aryl" refers to aryl groups further bearing one or more substituents. It should be understood that any combination term using an "ar" or "aryl" prefix refers to analogs according to the above definition of "aryl". For example, a term such as "aryloxy" refers to aryl group linked to a second group via an oxygen.

Each of the above defined "alkyl", "cycloalkyl", and "aryl" shall be understood to include their halogenated analogs, whereby the halogenated analogs may comprise one or several halogen atoms. The halogenated analogs thus comprise any halogen radical as defined in the following.

The term "halo" refers to a halogen radical selected from the group comprising fluoro, chloro, bromo and iodo. Preferred halo groups are fluoro, chloro and bromo.

The term "heteroaryl" refers to a stable 5 to 8 membered, preferably 5 or 6 membered monocyclic or 8 to 11 membered bicyclic aromatic heterocycle radical. Each heterocycle consists of carbon atoms and from 1 to 4 heteroatoms selected from the group consisting of nitrogen, oxygen, and sulfur. The heterocycle may be attached by any atom of the cycle which results in the creation of a stable structure. Preferred heteroaryl radicals as used herein include, for example, furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl, benzofuranyl, benzothienyl, indazolyl, benzimidazolyl, benzthiazolyl, benzoxazolyl, purinyl, quinolizinyl, quinolinyl, isoquinolinyl, cinnolinyl, phthalazinyl, quinazolinyl, quinoxalinyl, naphthridinyl, pteridinyl, carbazolyl, acridinyl, phenazinyl, phenothiazinyl and phenoxazinyl. "Substituted heteroaryl" refers to heteroaryl groups further bearing one or more substituents.

The term "heterocyclyl" refers to a stable 5 to 8 membered, preferably 5 or 6 membered monocyclic or 8 to 11 membered bicyclic heterocycle radical which may be either saturated or unsaturated, and is non-aromatic. Each heterocycle consists of carbon atom(s) and from 1 to 4 heteroatoms selected from the group consisting of nitrogen, oxygen and sulfur. The heterocycle may be attached by any atom of the cycle, which preferably results in the creation of a stable structure. Preferred heterocycle radicals as used herein include, for example, pyrrolinyl, pyrrolidinyl, pyrazolinyl, pyrazolidinyl, piperidinyl, morpholinyl, thiomorpholinyl, pyranyl, thiopyranyl, piperazinyl, indolinyl, azetidinyl, tetrahydropyranyl, tetrahydrothiopyranyl, tetrahydrofuranyl, hexahydropyrimidinyl, hexahydropyridazinyl, 1,4,5,6-tetrahydropyrimidin-2-ylamine, dihydro-oxazolyl, 1,2-thiazinanyl-1,1-dioxide, 1,2,6-thiadiazinanyl-1,1-dioxide, isothiazolidinyl-1,1-dioxide and imidazolidinyl-2,4-dione. "Mono―unsaturated heterocyclyl" refers to heterocyclyl containing one double bond or one triple bond. "Poly―unsaturated heterocyclyl" refers to heterocyclyl containing at least two double bonds or two triple bonds or a combination of at least one double bond and one triple bond.

"Substituted heterocyclyl" refers to heterocyclyl groups further bearing one or more substituents.

The terms "heterocyclyl", "heteroaryl" and "aryl", when associated with another moiety, unless otherwise specified, shall have the same meaning as given above. For example, "aroyl" refers to phenyl or naphthyl linked to a carbonyl group (C=O).

Each aryl or heteroaryl unless otherwise specified includes its partially or fully hydrogenated derivative. For example, quinolinyl may include decahydroquinolinyl and tetrahydroquinolinyl, naphthyl may include its hydrogenated derivatives such as tetrahydranaphthyl.

As used herein above and throughout this application, "nitrogen" or "N" and "sulfur" or "S" include any oxidized form of nitrogen such as nitrone, N-oxide and sulfur such as sulfoxide, sulfone and the quaternized form of any basic nitrogen such as HCl or TFA salts.

As used herein a wording defining the limits of a range of length such as e. g. "1 to 5" means any integer from 1 to 5, i. e. 1, 2, 3, 4 and 5. In other words, any range defined by two integers explicitly mentioned is meant to comprise any integer defining said limits and any integer comprised in said range.

As used herein the term "substituted" shall mean that one or more H atom of the group or compound which is substituted, is replaced by a different atom, a group of atoms, a molecule or a molecule moiety. Such atom, group of atoms, molecule or molecule moiety is also referred to herein as substituent.

As used herein the term "covalent bond" means a single, double or triple bond, preferably a single bond.

It will be understood by the ones skilled in the art that the kinin antagonist used in accordance with the present invention in its various embodiments and in particular when combined with a second pharmaceutically active agent, is preferably present in a pharmaceutical preparation or a pharmaceutical formulation. In connection therewith it is acknowledged that in case of a combination of two or more of such pharmaceutically active agents they can be present in a single pharmaceutical formulation or in different pharmaceutical formulations. In the latter case, there is preferably a hint or an instruaction in the package insert indicating that the formulation containing the first pharmaceutically active agent is preferably to be combined with a formulation containing the second or any further pharmaceutically active agent. Any of such or these formulation(s) may also be present or formed into a medicament. Apart from the pharmaceutically active agent(s) such formulation or medicament preferably comprises at least one pharmaceutically acceptable carrier, excipient or diluent.

In a further aspect the present invention is related to a method for the treatment of a patient suffering from bladder dysfunction as defined and described herein. Such method as well as the pharmaceutical composition or formulation comprises the administration of an effective or therapeutically effective amount of any kinin receptor antagonist or any combination comprising such kinin receptor antagonist as described herein. Preferably a patient is an organism suffering from any of the diseases described herein or being at risk to develop or suffer from such disease at a later point in time.

By an "effective" amount or a "therapeutically effective amount" of a drug or pharmacologically active agent is preferably meant a nontoxic but sufficient amount of the drug or agent to provide the desired effect, i.e., relieving the symptoms associated with bladder dysfunction, as explained above. It is recognized that the effective amount of a drug or pharmacologically active agent will vary depending on the route of administration, the selected compound, and the species to which the drug or pharmacologically active agent is administered, as well as the age, weight, and sex of the individual to which the drug or pharmacologically active agent is administered. It is also recognized that one of skill in the art will determine appropriate effective amounts by taking into account such factors as metabolism, bioavailability, and other factors that affect plasma levels of a drug or pharmacologically active agent following administration within the unit dose ranges disclosed further herein for different routes of administration.

It should be noted that the attending physician would know how to and when to terminate, interrupt, or adjust administration due to toxicity, to organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient. Typically, the dose will be between about 1-1000 mg/kg of body weight. About 1 mg to about 50 mg will preferably be administered to a child, and between 25 mg and about 1000 mg will preferably be administered to an adult.

For example, in the rat model of acetic-acid induced bladder irritation a dose of 1mg/kg (Icatibant, i.v.) was able to reverse the acetic acid induced detrusor instabilities. In view of this, a corresponding dosing and administration for human application is within the present invention. Apart from this particular regimen, it will be within the skills of the one of the art to determine, based on animal study results and in particular the one disclosed herein, suitable dosage and adminsitration regimens for the various bladder dysfunctions described herein for human application.

It will also be understood that the kinin receptor antagonist or any combination comprising such kinin receptor antagonist as described herein and used in accordance with the present invention is preferably present or used in a pharmaceutically acceptable or pharmacologically active form, although it is also within the present invention that either the kinin receptor antagonist and/or any pharmaceutically active agent used in connection therewith is present as a prodrug.

By "pharmaceutically acceptable," such as in the recitation of a "pharmaceutically acceptable carrier," or a "pharmaceutically acceptable acid addition salt," is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. "Pharmacologically active" (or simply "active") as in a "pharmacologically active" derivative or metabolite, refers to a derivative or metabolite having the same type of pharmacological activity as the parent compound. When the term "pharmaceutically acceptable" is used to refer to a derivative (e.g., a salt or an analog) of an active agent, it is to be understood that the compound is pharmacologically active as well, i.e., therapeutically effective for treating and/or alleviating bladder dysfunctions.

The term "physiologically tolerable salt" is preferably intended to mean a pharmaceutically acceptable salt that is not substantially toxic at the dosage administered to achieve the desired effect and does not independently possess significant pharmacological activity. The salts included within the scope of this term are hydrobromic, hydrochloric, sulfuric, phosphoric, nitric, formic, acetic, propionic, succinic, glycolic, lactic, malic, tartaric, citric, ascorbic, α-ketoglutaric, glutamic, aspartic, maleic, hydroxymaleic, pyruvic, phenylacetic, benzoic, p-aminobenzoic, anthranilic, p-hydroxybenzoic, salicyclic, hydroxyethanesulfonic, ethylenesulfonic, halobenzenesulfonic, toluenesulfonic, naphthalenesulfonic, methanesulfonic, sulfanilic, and the like.

Depending on the specific conditions being treated, such agents may be formulated and administrated systemically or locally. Techniques for formulation and administration may be found in "Remington's Pharmaceutical Sciences", 1990, 18th ed., Mack Publishing Co., Easton, PA. The administration of a compound according to the present invention can be done in a variety of ways, including, but not limited to, orally, intravesically, parenterally, subcutaneously, intramuscularly, intradermally, intravenously, intra-arterially, transdermally, intrathecally, transmucosally, bucally, lingually, sublingually, intranasally, rectally, urethrally, vaginally, or by inhalation, just to name a few.

The term "orally" is preferably used in its conventional sense to mean delivery of a drug through the mouth and ingestion through the stomach and digestive tract. The term "intravesically" is preferably used in its conventional sense to mean delivery of a drug directly into the bladder. By the term "parenterally" is preferably meant delivery by passage of a drug into the blood stream without first having to pass through the alimentary canal, or digestive tract. Parenteral administration may preferably be "subcutaneous", referring to delivery of a drug by administration under the skin. Another form of parenteral drug delivery is "intramuscular", preferably referring to delivery of a drug by administration into muscle tissue. Another form of parenteral drug delivery is "intradermal", preferably referring to delivery of a drug by administration into the skin. An additional form of parenteral drug delivery is "intravenous", preferably referring to delivery of a drug by administration into a vein. An additional form of parenteral drug delivery is "intra-arterial", preferably referring to delivery of a drug by administration into an artery. Another form of parenteral drug delivery is "transdermal", preferably referring to delivery of a drug by passage of the drug through the skin and into the bloodstream. Another form of parenteral drug delivery is "intrathecal", prerferably referring to delivery of a drug directly into the into the intrathecal space (where fluid flows around the spinal cord). Still another form of parenteral drug delivery is "transmucosal", preferably referring to administration of a drug to the mucosal surface of an individual so that the drug passes through the mucosal tissue and into the individual's blood stream. Transmucosal drug delivery may be "buccal" or "transbuccal", preferably referring to delivery of a drug by passage through an individual's buccal mucosa and into the bloodstream.

Another form of transmucosal drug delivery herein is "lingual" drug delivery, which preferabyly refers to delivery of a drug by passage of a drug through an individual's lingual mucosa and into the bloodstream. Another form of transmucosal drug delivery herein is "sublingual" drug delivery, which preferably refers to delivery of a drug by passage of a drug through an individual's sublingual mucosa and into the bloodstream. Another form of transmucosal drug delivery is "nasal" or "intranasal" drug delivery, referring to delivery of a drug through an individual's nasal mucosa and into the bloodstream. An additional form of transmucosal drug delivery herein is "rectal" or "transrectal" drug delivery, preferably referring to delivery of a drug by passage of a drug through an individual's rectal mucosa and into the bloodstream. Another form of transmucosal drug delivery is "urethral" or "transurethral" delivery, preferably referring to delivery of the drug into the urethra such that the drug contacts and passes through the wall of the urethra. An additional form of transmucosal drug delivery is "vaginal" or "transvaginal" delivery, preferably referring to delivery of a drug by passage of a drug through an individual's vaginal mucosa and into the bloodstream. An additional form of transmucosal drug delivery is "perivaginal" delivery, preferably referring to delivery of a drug through the vaginolabial tissue into the bloodstream.

The term "inhalation" is preferably used in its conventional sense to mean delivery of an aerosolized form of the drug by passage through the nose or mouth during inhalation and passage of the drug through the walls of the lungs.

Particularly preferred administrations of the kinin antagonists described herein is either local or systemic administration. Among the various forms of systemic administration, intravenous injection is particularly useful.

It will be acknowledged by the ones skilled in the art that the present invention may also be used in veterinary medicine. The exact dose will depend on the disorder to be treated and will be ascertainable by one skilled in the art using known techniques. A "patient" for the purposes of the present invention, i. e. to whom a compound or a pharmaceutical composition as defined herein is administered in accordance with the present invention, thus includes also animals. Thus the compounds, pharmaceutical compositions and methods are also applicable to or in connection with veterinary applications including diagnostic(s), diagnostic procedures and methods as well as staging procedures and methods. For example, the veterinary applications include, but are not limited to, canine, bovine, feline, porcine, caprine, equine, and ovine animals, as well as other domesticated animals including reptiles, such as iguanas, turtles and snakes, birds such as finches and members of the parrot family, lagomorphs such as rabbits, rodents such as rats, mice, guinea pigs, monkeys, hamsters, amphibians, fish, and arthropods. Valuable non-domesticated animals, such as zoo animals, may also be treated. In the preferred embodiment the patient is a mammal, and in the most preferred embodiment the patient is human.

It must be noted that as used in this specification and the appended embodiments, the singular forms "a," "an," and "the" include plural referents unless the context clearly dictates otherwise. Thus, for example, reference to "agonist" or "a pharmacologically active agent" includes a single active agent as well as two or more different active agents in combination.

The present invention will now be further illustrated by the following figures, examples and the sequence listing from which further features, embodiments and advantageous may be taken ,whererby
Fig 1 shows a diagram indicating the dose-dependent effect of Icatibant on the intercontraction interval (ICI) at different time points after start of acetic acid infusion in comparison with vehicle and positive control (Aspirin);
Fig. 2 shows a diagram indicating the dose dependent effect of Icatibant on the bladder capacity at different time points after start of acetic acid infusion in comparison with vehicle and positive control (Aspirin); and
Fig. 3 shows a diagramm indicating the effect of Icatibant on the micturition pressure at different time points after start of acetic acid infusion in comparison with vehicle and positive control (Aspirin).

The abbreviations used herein for amino acids correspond to the three letter code customary in peptide chemistry as is described in Eur. J. Biochem 138, 9 (1984). Other abbreviations used are listed below:
- Aeg: N-(2-Aminoethyl)glycine
- Cpg: Cyclopentylglycyl
- Fmoc: 9-Fluorenylmethyloxycarbonyl
- Nal: 2-Naphthylalanyl
- Oic: cis,endo-Octahydroindole-2-carbonyl
- Thi: 2-Thienylalanyl
- Tic: 1,2,3,4- Tetrahydroisoquinoline-3-ylcarbonyl
- Hyp: trans-4-hydroxy-L-proline
- D-HypE(transpropyl): trans-4-propyloxy-D-proline
- Arg(Tos): 2-amino-5-(N-tosyl)guanidinopentanoic acid
- Tos or tosyl: 4-toluenesulfony
- Aoc: cis,endo-2-azabicyclo[3.3.0]octane-3-S-carbonyl
- Aeg(Fmoc): N-(2-(Fmoc)aminoethyl)glycine
- Ac: acetyl
- Orn: ornithine
- Igl: alpha-(2-indanyl)glycine
- F5F: pentafluorophenylalanine
- Ala: alanine
- Cys: cysteine
- Asp: aspartic acid
- Glu: glutamic acid
- Phe: phenylalanin
- Gly: glycine
- His: histidine
- Ile: isoleucine
- Lys: lysine
- Leu: leucin
- Met: methionine
- Asn: asparagine
- Pro: proline
- Gln: glutamine
- Arg: arginine
- Ser: serine
- Thr: threonine
- Val: valine
- Trp: tryptophan
- Tyr: tyrosine

### Example 1: The effect of Icatibant on rat model of acetic acid bladder irritation

### Objective and Rationale

The objective of this study was to determine the ability of Icatibant to improve bladder function in the rat model of acetic acid induced bladder irritation, a commonly used model of Overactive Bladder (OAB, Shinozaki et al., Biomed Res. 2005 Feb;26(1):29-33; Mitsui et al., J Neurophysiol. 2001 Nov;86(5):2276-84; Yu & de Groat, Brain Res. 1998 Oct 5;807(1-2):11-8; Oki et al., Journal of Urology. 173(4):1395-1399, April 2005, Atala, A., Journal of Urology. 170(5):1701-1702, November 2003). In particular, the current study utilized Icatibant as an exemplary B2 kinin receptor antagonist and characterized its effects on bladder capacity, intercontraction interval (voiding frequency) and micturition pressure.

### Materials and Methods

Groups of 5 female Wistar rats weighing 250 to 300 g anesthetized with urethane 1.2 g/kg i.p. in 5 ml/kg were used. A polyethylene catheter (PE50) was implanted into the bladder for saline or acetic acid infusion through a 3 way stopcock to which a pressure transducer is connected for measurements of bladder pressure (mm Hg). Saline at 37°C was infused into the bladder at a constant rate until cystometrography becomes stable. Thereafter, instillation of 0.2 % acetic acid into the urinary bladder was initiated. Icatibant (1 mg/kg), vehicle (1 ml/kg) as well as positive control (Aspirin, 10 mg/kg) were administered intravenousely (IV) via a PE-10 catheter in a femoral vein 5 minutes after the first micturition cycle following the start of acetic acid infusion. The following parameters were measured continuously for 1 hour after treatment was started: intercontraction interval (ICI, minutes, defined as the time between two voiding cycles), threshold pressure (mmHg), bladder capacity (ml), micturition volume (ml), and micturition pressure (mmHg). Micturition volume is derived from the weight of excreted urine measured by a balance placed below the rat.

### Data Analysis

Paired Student's test was applied for comparison between the time before and after test substance or with vehicle treatment. To signify differences between test substance and vehicle control group, Dunnett's test was used. Differences are considered significant at P<0.05.

### Results and Conclusions

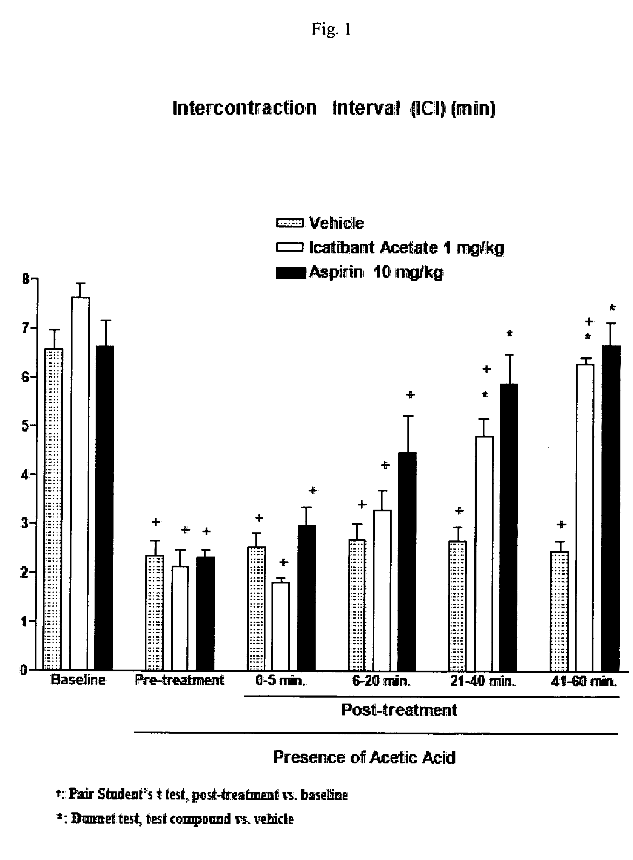

The effect of Icatibant on the intercontraction interval (ICI) at different time points after start of acetic acid infusion in comparison with vehicle and positive control (Aspirin) is depicted in Fig.1. The intercontraction interval in the Icatibant treated group dose-dependently increases in comparison to the vehicle group.

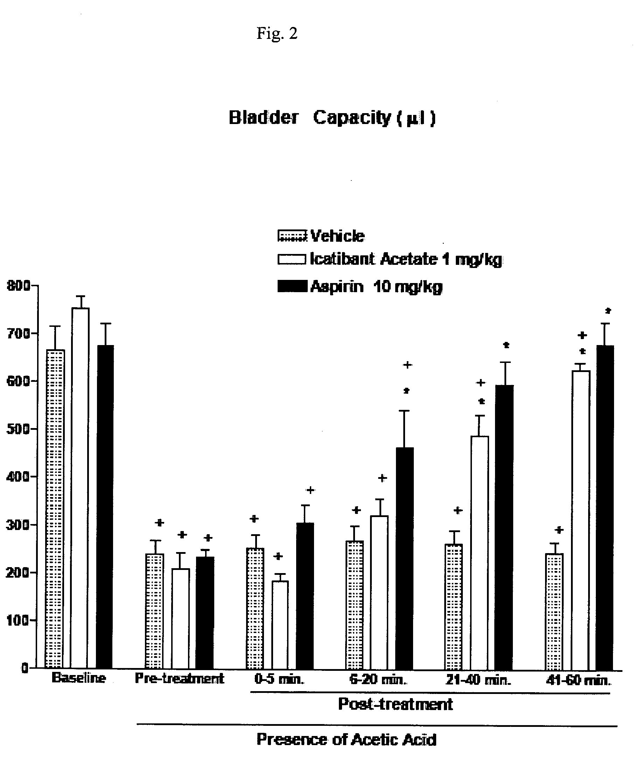

The effect of Icatibant on the bladder capacity at different time points after start of acetic acid infusion in comparison with vehicle and positive control (Aspirin) is depicted in Fig.2. The bladder capacity dose-dependently increases in the Icatibant treated group in comparison to the vehicle group.

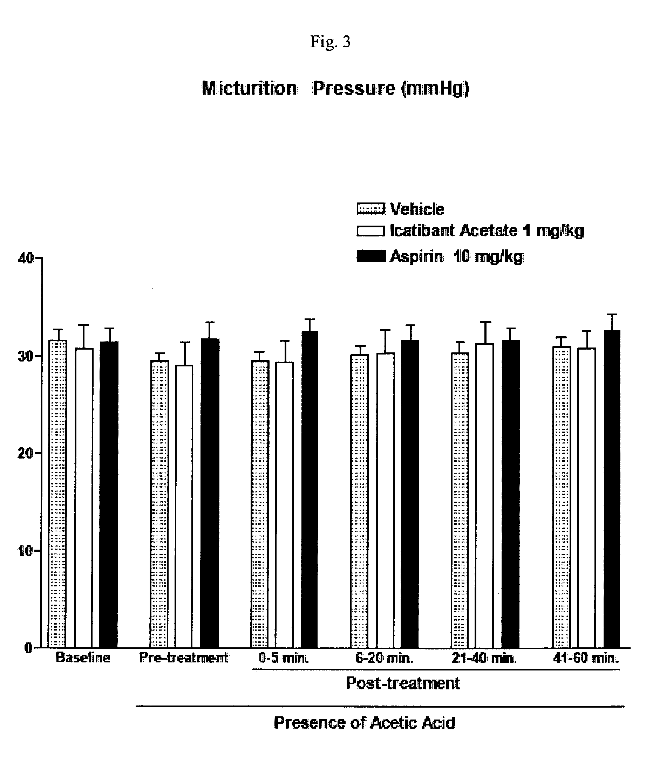

The effect of Icatibant on the micturition pressure at different time points after start of acetic acid infusion in comparison with vehicle and positive control (Aspirin) is depicted in Fig.3. There is no decrease in micturition pressure, indicating no risk for development of urine retiontion syndroms for Icatibant treatment in comparison to anticholinergics.

### Summary

Icatibant, almost in the relatively low dose of 1mg/kg, was capable of doubling the interval between two micturition contractions and bladder capacity, without any change of micturition pressure. These findings strongly indicate efficacy of kinin B2 receptor antagonists for detrusor instabilities associated with overactive bladder (OAB) in human patients.

The features of the present invention disclosed in the specification, the claims, the sequence listing and/or the drawings may both separately and in any combination thereof be material for realizing the invention in various forms thereof.

## Claims

1. Use of a kinin receptor antagonist for the manufacture of a medicament for the treatment and/or prevention of bladder dysfunction, whereby the kinin receptor is selected from the group comprising B1 and B2 receptors.

2. Use according to claim 1, whereby the kinin receptor is the B1 receptor.

3. Use according to claim 2, whereby the kinin receptor antagonist is selected from the group comprising
Ac-Lys-Arg-Pro-Pro-Gly-Phe-Ser-D-Nal-Ile-OH (SEQ. ID NO. 1),
Ac-Lys-Arg-Pro-Pro-Gly-N-MePhe-Ser-D-Nal-Ile-OH (SEQ. ID NO. 2),
Ac-Lys-Lys-Arg-Pro-Pro-Gly-NMePhe-Ser-D-Nal-Ile-OH (SEQ. ID NO. 3),
Ac-Orn-Arg-Oic-Pro-Gly-NMePhe-Ser-D-Nal-Phe-OH (SEQ. ID NO. 4),
H-Lys-Lys-Arg-Pro-Hyp-Gly-Igl-Ser-D-Igl-Oic-OH (SEQ. ID NO. 5),
H-Lys-Lys-Arg-Pro-Hyp-Gly-Cpg-Ser-D-Tic-Cpg-OH (SEQ. ID NO. 6),
2-[1-(3,4-Dichloro-benzene sulfonyl)-3-oxo-1,2,3,4-tetrahydro-chinoxaline-2-yl]-N-{2-[4-(4,5-dihydro-1H-imidazole-2-yl)-phenyl]-ethyl}-acetamide,
N- {2-[4-(4,5-Dihydro-1H-imidazole-2-yl)-phenyl]-ethyl}-2-[1-(naphthaline-2-sulfonyl)-3-oxo-1,2,3,4-tetrahydro-chinoxaline-2-yl]-acetamide,
3-(3,4-Dichloro-phenyl)-N- {1-[4-(4,5-dihydro-1H-imidazole-2-yl)-benzyl]-2-oxo-2-pyrrolidine-1-yl-ethyl}-3-(naphthaline-2-sulfonyl amino)-propionamide,
4'-(1-{3-[(2,2-Difluoro-cyclopropane carbonyl)-amino]-4-methyl-pyridine-2-ylamino}-ethyl)-5-methyl-biphenyl-2-carboxylic acid methyl ester,
N-(4-Chloro-2-{1-[3'-fluoro-2'-(3-methyl-[1,2,4]oxadiazole-5-yl)-biphenyl-4-yl]-ethylamino}-pyridine-3-yl)-3,3,3-trifluoro propionamide,
3-Benzo[1,3]dioxol-5-yl-N-[2-[4-(2,6-dimethyl-piperidine-1-yl methyl)-phenyl]-1-(isopropyl-methyl-carbamoyl)-ethyl]-3-(6-methoxy-naphthaline-2-sulfonyl amino)-propionamide,
{2-(2,2-Diphenyl-ethylamino)-5-[4-(4-isopropyl-piperazine-1-carbonyl)-piperidine-1-sulfonyl]-phenyl}-morpholine-4-yl-methanone, and
{2-(2,2-Diphenyl-ethylamino)-5-[4-(4-methyl-piperazine-1-carbonyl)-piperidine-1-sulfonyl]-phenyl}-morpholine-4-yl-methanone.

4. Use according to claim 1, whereby the kinin receptor is the B2 receptor.

5. Use according to claim 4, whereby the kinin receptor antagonist is selected from the group comprising
MEN 11270,
H-D-Arg-Arg-Pro-Hyp-Gly-Igl-Ser-D-F5F-Igl-Arg-H (SEQ. ID NO. 7),
H-Arg-Arg-Pro-Hyp-Gly-Igl-Ser-D-Igl-Oic-Arg-OH (SEQ. ID NO. 8),
[H-D-Arg-Arg-Pro-Hyp-Gly-Phe-Cys-D-Phe-Leu-Arg-H]₂BSH (SEQ. ID NO. 9),
4-{2-[({[3-(3-Brom-2-methyl-imidazo[1,2-a]pyridine-8-yl oxymethyl)-2,4-dichlore-phenyl]-methyl-carbamoyl}-methyl)-carbamoyl]-vinyl}-N,N-dimethyl-benzamide,
3-(6-Acetylamino-pyridine-3-yl)-N-({[2,4-dichloro-3-(2-methyl-chinoline-8-yl oxymethyl)-phenyl]-methyl-carbamoyl}-methyl)-acrylamide,
1-[2,4-Dichloro-3-(2,4-dimethyl-chinoline-8-yl oxymethyl)-benzene sulfonyl]-pyrrolidine-2-carboxylic acid [3-(4-carbamimidoyl-benzoylamino)-propyl]-amide,
Bradizide,
4-(4-{1-[2,4-Dichloro-3-(2,4-dimethyl-chinoline-8-yl oxymethyl)-benzene sulfonyl]-pyrrolidine-2-carbonyl}-piperazine-1-carbonyl)-benzamidine, and
2-[5-(4-Cyano-benzoyl)-1-methyl-1H-pyrrol-2-yl]-N-[2,4-dichloro-3-(2-methyl-chinoline-8-yloxymethyl)-phenyl]-N-methyl-acetamide.

6. Use according to claim 4, whereby the kinin receptor antagonist is a peptide of the formula (I)
**Z-P-A-B-C-E-F-K-(D)Q-G-M-F'-I** (I)
in which:
Z is a₁) hydrogen, (C₁-C₈)-alkyl, (C₁-C₈)-alkanoyl, (C₁-C₈)-alkoxycarbonyl, (C₃-C₈)-cycloalkyl, (C₄-C₉) ―cycloalkanoyl or (C₁-C₈)-alkylsulfonyl,
in which 1, 2 or 3 hydrogen atoms in each case are optionally individually and independent from each other replaced by 1, 2 or 3 identical or different radicals selected from the group consisting of carboxyl, NHR (1), [(C₁-C₄)-alkyl]NR(1) or [(C₆-C₁₀)-aryl-(C₁-C₄)-alkyl]NR(1), where R(1) is hydrogen or a urethane protective group, (C₁-C₄)-alkyl, (C₁-C₈)-alkylamino, (C₆-C₁₀)-aryl-(C₁-C₄)-alkylamino, hydroxyl, (C₁-C₄)-alkoxy, halogen, di-(C₁-C₈)-alkylamino, di-[(C₆-C₁₀)-aryl-(C₁-C₄)]-alkylamino, carbamoyl, phthalimido, 1,8-naphthalimido, sulfamoyl, (C₁-C₄) alkoxycarbonyl, (C₆-C₁₄)-aryl and (C₆-C₁₄)-aryl-(C₁-C₅)-alkyl,
or in which 1 hydrogen atom in each case is optionally replaced by a radical selected from the group consisting of (C₃-C₈)-cycloalkyl, (C₁-C₆)-alkylsulfonyl, (C₁-C₆)-alkylsulfinyl, (C₆-C₁₄)-aryl-(C₁-C₄)-alkylsulfonyl, (C₆-C₁₄)-aryl-(C₁-C₄)-alkylsulfinyl, (C₆-C₁₄)-aryl, (C₆-C₁₄)-aryloxy, (C₃-C₁₃)-heteroaryl and (C₃-C₁₃)- heteroaryloxy,
and 1 or 2 hydrogen atoms are replaced by 1 or 2 identical or different radicals selected from the group consisting of carboxyl, amino, (C₁-C₈)-alkylamino, hydroxyl, (C₁-C₄)-alkoxy, halogen, di-(C₁-C₈)-alkylamino, carbamoyl, sulfamoyl, (C₁-C₄)-alkoxycarbonyl, (C₆-C₁₄)-aryl and (C₆-C₁₄)-aryl-(C₁-C₅)-alkyl;
a₂) (C₆-C₁₄)-aryl, (C₇-C₁₅)-aroyl, (C₆-C₁₄)-arylsulfonyl, (C₃-C₁₃)-heteroaryl or (C₃-C₁₃)-heteroaroyl; or
a₃) carbamoyl which can optionally be substituted on the nitrogen by (C₁-C₈)-alkyl, (C₆-C₁₄)-aryl or (C₆-C₁₄)-aryl-(C₁-C₅)-alkyl;
where in the radicals defined under a₁), a₂) and a₃) the aryl, heteroaryl, aroyl, arylsulfonyl and heteroaroyl groups are optionally substituted by 1, 2, 3 or 4 radicals individually and independently selected from the group consisting of carboxyl, amino, nitro, (C₁-C₈)-alkylamino, hydroxyl, (C₁-C₆)-alkyl, (C₁-C₆)-alkoxy, (C₆-C₁₄)-aryl, (C₇-C₁₅)-aroyl, halogen, cyano, di-(C₁-C₈)-alkylamino, carbamoyl, sulfamoyl and (C₁-C₆)-alkoxycarbonyl;
**P** is a covalent bond or a radical of the formula II,
-NR(2)―(U)―CO- (II)
in which
R(2) is hydrogen, methyl or a urethane protective group,
U is (C₃-C₈)-cycloalkylidene, (C₆-C₁₄)-arylidene, (C₃-C₁₃)-heteroarylidene, (C₆-C₁₄)-aryl-(C₁-C₆)-alkylidene, which optionally individually and independent from each other can be substituted, or [CHR(3)]ₙ,
where n is any integer from 1-8, preferably any integer from 1-6,
any R(3) is independently and individually selected from the group comprising hydrogen, (C₁-C₆)-alkyl, (C₃-C₈)-cycloalkyl, (C₆-C₁₄)-aryl, (C₃-C₁₃)-heteroaryl, whereby under the proviso that R(3) is different from hydrogen, (C₁-C₆)-alkyl, (C₃-C₈)-cycloalkyl, (C₆-C₁₄)-aryl and (C₃-C₁₃)-heteroaryl are optionally monosubstituted by amino, substituted amino, amidino, substituted amidino, hydroxyl, carboxyl, carbamoyl, guanidino, substituted guanidino, ureido, substituted ureido, mercapto, methylmercapto, phenyl, 4-chlorophenyl, 4-fluorophenyl, 4-nitrophenyl, 4-methoxyphenyl, 4-hydroxyphenyl, phthalimido, 1,8-naphthalimido, 4-imidazolyl, 3-indolyl, 2-thienyl, 3-thienyl, 2-pyridyl, 3-pyridyl or cyclohexyl, or
in which R(2) and R(3), together with the atoms carrying them, form a mono-, bi- or tricyclic ring system having 2 to 15 carbon atoms;
**A** is defined as P;
**B** is a basic amino acid in the L-or D-configuration, which can be substituted in the side chain;
**C** is a compound of the formula III a or III b 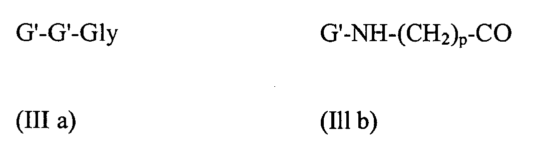 in which
p is any integer from 2 to 8, and
any G' is independently a radical of the formula IV
-NR(4)-CHR(5)-CO- (IV)
in which
R(4) and R(5), together with the atoms carrying them, form a heterocyclic mono-, bi-or tricyclic ring system having 2 to 15 carbon atoms;
**E** is a radical of a neutral, acidic or basic, aliphatic or alicyclic-aliphatic amino acid;
**F** independently of one another is a radical of a neutral, acidic or basic, aliphatic or aromatic amino acid which can be substituted in the side chain, or a covalent bond;
**(D)Q** is D-Tic, D-Phe, D-Oic, D-Thi or D-Nal, any of which is optionally substituted by halogen, methyl or methoxy or is a radical of the formula (V) below 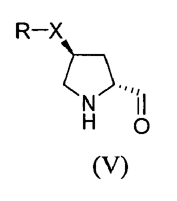 in which
X is oxygen, sulfur or a covalent bond;
R is hydrogen, (C₁-C₈)-alkyl, (C₃-C₈)-cycloalkyl, (C₆-C₁₄)-aryl, (C₆-C₁₄)-aryl-(C₁-C₄)-alkyl, where the alicyclic system can optionally be substituted by halogen, methyl or methoxy;
**G** is defined as G' above or is a covalent bond;
**F'** is defined as F, is a radical -NH-(CH₂)_{q}-, where q=2 to 8, or, if G is not a direct bond, is a direct bond;
**I** is -OH, -NH₂ or NHC₂H₅;
**K** is the radical -NH-(CH₂)ₓ-CO-, where x=1-4, or is a covalent bond, and
**M** is defined as F,
or a physiologically tolerable salt thereof.

7. Use according to claim 6, whereby the peptide is a peptide of formula I wherein
Z is as defined above under a₁), a₂) or a₃), preferably hydrogen
**P** is a covalent bond or a radical of the formula II
-NR(2)-(U)―CO― (II)
where
U is CHR(3),
R(3) is as defined above, and
R(2) is H or CH₃,
**A** is a covalent bond.
or a physiologically tolerable salt thereof.

8. Use according to claim 6 or 7, preferably claim 7,
whereby
**Z** is as defined above under a₁), a₂) or a₃), preferably hydrogen
**P** is a covalent bond or a radical of the formula II
-NR(2)―(U)-CO- (II)
where
U is CHR(3) and
R(3) is individually and independently selected from the group comprising hydrogen, (C1-C6)-alkyl, (C3-C8)-cycloalkyl, (C6-C14)-aryl, (C3-C13)-heteroaryl, whereby under the proviso that R(3) is different from hydrogen, (C₁-C₆)-alkyl, (C₃-C₈)-cycloalkyl, (C₆-C₁₄)-aryl and (C₃-C₁₃)-heteroaryl are optionally monosubstituted by amino, substituted amino, hydroxyl, carboxyl, carbamoyl, guanidino, substituted guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pyridyl or cyclohexyl,
or in which R(2) and R(3), together with the atoms carrying them, form a mono-, bi-or tricyclic ring system having 2 to 15 carbon atoms,
R(2) is H or CH3;
**A** is a covalent bond;
**(D)Q** is D-Tic.

9. Use according to any of claims 6 to 8 preferably 8, whereby the peptide is
H-D-Arg-Arg-Pro-Hyp-Gly-Thi-Ser-D-Tic-Oic-Arg-OH (SEQ. ID NO. 10),
para-guanidobenzoyl-Arg-Pro-Hyp-Gly-Thi-Ser-D-Tic-Oic-Arg-OH (SEQ. ID NO. 11),
H-D-Arg-Arg-Pro-Hyp-Gly-Phe-Ser-D-HypE(transpropyl)-Oic-Arg-OH (SEQ. ID NO. 12),
H-D-Arg-Arg-Pro-Hyp-Gly-Cpg-Ser-D-Cpg-Cpg-Arg-OH (SEQ. ID NO. 13),
H-D-Arg-Arg-Pro-Pro-Gly-Thi-Ser-D-Tic-Oic-Arg-OH (SEQ. ID NO. 14),
H-Arg(Tos)-Pro-Hyp-Gly-Thi-Ser-D-Tic-Oic-Arg-OH (SEQ. ID NO. 15),
H-Arg(Tos)-Pro-Hyp-Gly-Phe-Ser-D-Tic-Oic-Arg-OH (SEQ. ID NO. 16),
H-D-Arg -Arg-Pro-Hyp-Gly-Phe-Ser-D-Tic-Oic-Arg-OH (SEQ. ID NO. 17),
Fmoc-D-Arg-Arg-Pro-Hyp-Gly-Thi-Ser-D-Tic-Oic-Arg-OH (SEQ. ID NO. 18),
Fmoc-Aoc-D-Arg-Arg-Pro-Hyp-GIy-Thi-Ser-D-Tic-Oic-Arg-OH (SEQ. ID NO. 19),
Fmoc-ε-aminocaproyl-D-Arg-Arg-Pro-Hyp-Gly-Thi-Ser-D-Tic-Oic-Arg-OH (SEQ. ID NO. 20),
benzoyl-D-Arg-Arg-Pro-Hyp-Gly-Thi-Ser-D-Tic-Oic-Arg-OH (SEQ. ID NO. 21),
cyclohexylcarbonyl-D-Arg-Arg-Pro-Hyp-Gly-Thi-Ser-D-Tic-Oic-Arg-OH (SEQ. ID NO. 22),
Fmoc-Aeg(Fmoc)-D-Arg-Arg-Pro-Hyp-Gly-Thi-Ser-D-Tic-Oic-Arg-OH (SEQ. ID NO. 23),
Fmoc-Aeg(Fmoc)-Arg-Pro-Hyp-Gly-Thi-Ser-D-Tic-Oic-Arg-OH (SEQ. ID NO. 24),
indol-3-yl-acetyl-D-Arg-Arg-Pro-Hyp-Gly-Thi-Ser-D-Tic-Oic-Arg-OH (SEQ. ID NO. 25),
dibenzylacetyl-D-Arg-Arg-Pro-Hyp-Gly-Thi-Ser-D-Tic-Oic-Arg-OH (SEQ. ID NO. 26),
or a physiologically tolerable salt thereof.

10. Use according to claim 9, wherein the peptide is
H-D-Arg-Arg-Pro-Hyp-Gly-Thi-Ser-D-Tic-Oic-Arg-OH (SEQ. ID NO. 10) or
para-guanidobenzoyl-Arg-Pro-Hyp-Gly-Thi-Ser-D-Tic-Oic-Arg-OH (SEQ. ID NO.11); preferably
H-D-Arg-Arg-Pro-Hyp-Gly-Thi-Ser-D-Tic-Oic-Arg-OH (HOE 140) (SEQ. ID NO. 10)
or a physiologically tolerated salt thereof.

11. Use according to any of claims 1 to 10, whereby the bladder dysfunction is associated with one or more disease patterns selected from the group comprising urinary frequency, urinary urgency, dysuria, urinary incontinence, enuresis, loss of bladder function, and nocturia.

12. Use according to claim 11, whereby the bladder dysfunction is associated with urinary frequency.

13. Use according to claim 11, whereby the bladder dysfunction is associated with urinary urgency.

14. Use according to claim 11, whereby the bladder dysfunction is associated with urinary incontinence.

15. Use according to any of claim 1 to 14, whereby the bladder dysfunction is correlated with and/or caused by one or more disorders selected from the group comprising neurogenic, myogenic, neoplastic, preferably benign prostatic hyperplasia, inflammatory, metabolic and idiopathic disorders, prostate hypertrophy, hormone dysregulation, bladder obstruction due to surgery or injury, anatomical changes of the urogenital tract, multiple sclerosis, parkinson's disease, stroke, diabetes and aging.

16. Use according to claim 15, whereby the bladder dysfunction is correlated with and/or caused by neurogenic disorders.

17. Use according to claim 15, whereby the bladder dysfunction is correlated with and/or caused by myogenic and/or inflammatory disorders.

18. Use according to claim 15 , whereby the bladder dysfunction is correlated with and/or caused by idiopathic disorders.

19. Use according to claim 15, whereby the bladder dysfunction is correlated with and/or caused by neoplastic and/or metabolic disorders and/or is associated with diabetes or aging.

20. Use according to any of claims 1 to 19, whereby the bladder dysfunction is correlated with C-fibre activation and/or sensitization, preferably pathological C-fibre activation and/or sensitization.

21. Use according to any of claims 1 to 20, whereby the bladder dysfunction and the correlated and/or causing disease patterns cannot be treated or prevented by a compound selected from the group comprising cholinergic antagonists, adrenergic antagonists, adrenergic agonists, vasopressin agonists, neurokinin antagonists, potassium channel activators, analgesics, NO donors, Ca²⁺ modulators, spasmolytics, muscle relaxants, preferably botulinus toxin, 5HT re-uptake inhibitors, purinergic receptor antagonists, PDE inhibitors, and VR1 modulators.

22. Use according to any of claims 1 to 21, whereby the medicament is to be administered to a patient in need thereof in a therapeutically effective amount.

23. Use according to any of claims 1 to 22, whereby the medicament is present in an oral dosage form, whereby such oral dosage form is selected from the group comprising powder, preferably a dispersible powder, capsule, tablet, solution, and liquid suspension.

24. Use according to any of claims 1 to 22, whereby the medicament is for parenteral administration

25. Use according to any of claims 1 to 22, whereby the medicament is for local and/or systemic administration.

26. Use according to any of claims 1 to 22, whereby the medicament is for administration to the patient by a route selected from the group comprising intravenous, subcutaneous, intravesical, intramuscular, intrathecal, nasal, rectal, sublingual, transurethral, intravaginal perivaginal, intraperitoneal, transmucosal, transdermal administration and inhalation.

27. Use according to any of claims 1 to 26, whereby the medicament comprises at least one further pharmaceutically active compound.

28. Use according to claim 27, whereby the further pharmaceutically active compound is selected from the group comprising cholinergic antagonists, adrenergic antagonists or agonists, vasopressin agonists, neurokinin antagonists, potassium channel activators, analgesics, NO donors, Ca²⁺ modulators, spasmolytics, muscle relaxants, preferably botulinus toxin, 5HT re-uptake inhibitors, purinergic receptor antagonists, PDE inhibitors, and VR1 modulators.

29. Use according to any of claims 1 to 28, wherein the kinin antagonist and/or at least one further pharmaceutically active compound is/are present as a pharmaceutically acceptable salt, ester, amides, prodrug, or a pharmaceutically active solvate.

30. Use according to any of claims 1 to 29, whereby the kinin antagonist is metabolized to one or more pharmaceutically active molecules.

31. Use according to any of claims 1 to 30, whereby the medicament comprises a pharmaceutically acceptable carrier, diluent or excipient.

32. Use according to any of claims 1 to 31, whereby the medicament comprises a multitude of individualised dosages and/or administration forms.

33. Use according to any of claims 1 to 32, whereby the medicament is used for the treatment and/or prevention of bladder dysfunction in animals.


SEQUENCE LISTING

<110>  Jerini AG

<120>  Use of kinin antagonists

<130>  J 10036 EP

<160>  26

<170>  PatentIn version 3.1

<210>  1
<211>  9
<212>  PRT
<213>  Artificial

<220>
<221>  MISC_FEATURE
<222>  (8)..(8)
<223>  X is D-Nal

<400>  1

Lys Arg Pro Pro Gly Phe Ser Xaa Ile
1               5

<210>  2
<211>  9
<212>  PRT
<213>  Artificial

<220>
<221>  MISC_FEATURE
<222>  (6)..(6)
<223>  X is N-MePhe

<220>
<221>  MISC_FEATURE
<222>  (8)..(8)
<223>  X is D-Nal

<400>  2

Lys Arg Pro Pro Gly Xaa Ser Xaa Ile
1               5

<210>  3
<211>  10
<212>  PRT
<213>  Artificial

<220>
<221>  MISC_FEATURE
<222>  (7)..(7)
<223>  X is NMePhe

<220>
<221>  MISC_FEATURE
<222>  (9)..(9)
<223>  X is D-Nal

<400>  3

Lys Lys Arg Pro Pro Gly Xaa Ser Xaa Ile
1               5                   10

<210>  4
<211>  9
<212>  PRT
<213>  Artificial

<220>
<221>  MISC_FEATURE
<222>  (1)..(1)
<223>  X is Orn

<220>
<221>  MISC_FEATURE
<222>  (3)..(3)
<223>  X is Oic

<220>
<221>  MISC_FEATURE
<222>  (6)..(6)
<223>  X is NmePhe

<220>
<221>  MISC_FEATURE
<222>  (8)..(8)
<223>  X is D-Nal

<400>  4

Xaa Arg Xaa Pro Gly Xaa Ser Xaa Phe
1               5

<210>  5
<211>  10
<212>  PRT
<213>  Artificial

<220>
<221>  MISC_FEATURE
<222>  (5)..(5)
<223>  X is Hyp

<220>
<221>  MISC_FEATURE
<222>  (7)..(7)
<223>  X is Igl

<220>
<221> MISC_FEATURE
<222> (9)..(9)
<223> D-Igl

<220>
<221> MISC_FEATURE
<222> (10)..(10)
<223> Oic

<400> 5

Lys Lys Arg Pro Xaa Gly Xaa Ser Xaa Xaa
1               5                   10

<210> 6
<211> 10
<212> PRT
<213> Artificial

<220>
<221> MISC_FEATURE
<222> (5)..(5)
<223> X is Hyp

<220>
<221> MISC_FEATURE
<222> (7)..(7)
<223> X is Cpg

<220>
<221> MISC_FEATURE
<222> (9)..(9)
<223> X is D-Tic

<220>
<221> MISC_FEATURE
<222> (10)..(10)
<223> X is Cpg

<400> 6

Lys Lys Arg Pro Xaa Gly Xaa Ser Xaa Xaa
1               5                   10

<210> 7
<211> 10
<212> PRT
<213> Artificial

<220>
<221> MISC_FEATURE
<222> (1)..(1)
<223> X is D-Arg

<220>
<221>  MISC_FEATURE
<222>  (4)..(4)
<223>  X is Hyp


<220>
<221>  MISC_FEATURE
<222>  (6)..(6)
<223>  X is Igl


<220>
<221>  MISC_FEATURE
<222>  (8)..(8)
<223>  X is D-F5F


<220>
<221>  MISC_FEATURE
<222>  (9)..(9)
<223>  X is Igl


<400>  7

Xaa Arg Pro Xaa Gly Xaa Ser Xaa Xaa Arg
1               5                   10


<210>  8
<211>  9
<212>  PRT
<213>  Artificial

<220>
<221>  MISC_FEATURE
<222>  (4)..(4)
<223>  X is Hyp


<220>
<221>  MISC_FEATURE
<222>  (6)..(6)
<223>  X is Igl


<220>
<221>  MISC_FEATURE
<222>  (8)..(8)
<223>  X is D-Igl


<220>
<221>  MISC_FEATURE
<222>  (9)..(9)
<223>  X is Oic


<400>  8

Arg Arg Pro Xaa Gly Xaa Ser Xaa Arg
1               5

<210>  9
<211>  10
<212>  PRT
<213>  Artificial

<220>
<221>  MISC_FEATURE
<222>  (1)..(1)
<223>  X is D-Arg

<220>
<221>  MISC_FEATURE
<222>  (4)..(4)
<223>  X isHyp

<220>
<221>  MISC_FEATURE
<222>  (8)..(8)
<223>  X is D-Phe

<220>
<221>  MISC_FEATURE
<222>  (1)..(10)
<223>  two of the peptides bound to BSH

<400>  9

Xaa Arg Pro Xaa Gly Phe Cys Xaa Leu Arg
1               5                   10

<210>  10
<211>  10
<212>  PRT
<213>  Artificial

<220>
<221>  MISC_FEATURE
<222>  (1)..(1)
<223>  X is an arginine analog in the D configuration

<220>
<221>  MISC_FEATURE
<222>  (4)..(4)
<223>  X is a 4-hydroxyproline moiety

<220>
<221>  MISC_FEATURE
<222>  (6)..(6)
<223>  X is a 2-thienylalanyl moiety

<220>

<221>  MISC_FEATURE
<222>  (8)..(8)
<223>  X is 1,2,3,4-tetrahydroisoquinoline-3-yl carbony-moiety in the D-
       configuration


<220>
<221>  MISC_FEATURE
<222>  (9)..(9)
<223>  X is  acis, endo-octahydroindole-2-carbonyl moiety


<400>  10

Xaa Arg Pro Xaa Gly Xaa Ser Xaa Xaa Arg
1               5                   10


<210>  11
<211>  9
<212>  PRT
<213>  Artificial

<220>
<221>  MISC_FEATURE
<222>  (1)..(1)
<223>  X is an arginine analog N-substituted with p-guanidobenzoyl


<220>
<221>  MISC_FEATURE
<222>  (3)..(3)
<223>  X is a 4-hydroxyproline moiety


<220>
<221>  MISC_FEATURE
<222>  (5)..(5)
<223>  X is a 2-thienylalanyl moiety


<220>
<221>  MISC_FEATURE
<222>  (7)..(7)
<223>  X is a 1,2,3,4 - tetrahydroisoquinoline-3-yl carbonyl moiety in t
       he D-configuration


<220>
<221>  MISC_FEATURE
<222>  (8)..(8)
<223>  X is a cis, endo-octahydroindole-2-carbonyl moiety


<400>  11

Xaa Pro Xaa Gly Xaa Ser Xaa Xaa Arg
1               5


<210>  12
<211>  10
<212>  PRT

<213>  Artificial

<220>
<221>  MISC_FEATURE
<222>  (1)..(1)
<223>  X is arginine in the D-configuration


<220>
<221>  MISC_FEATURE
<222>  (4)..(4)
<223>  X is a 4-hydroxyproline moiety


<220>
<221>  MISC_FEATURE
<222>  (8)..(8)
<223>  X is a trans-4-propyloxyproline in the D-configuration


<220>
<221>  MISC_FEATURE
<222>  (9)..(9)
<223>  X is a cis,endo-octahydroindole-2-carbonyl moiety


<400>  12

Xaa Arg Pro Xaa Gly Phe Ser Xaa Xaa Arg
1               5                   10


<210>  13
<211>  10
<212>  PRT
<213>  Artificial

<220>
<221>  MISC_FEATURE
<222>  (1)..(1)
<223>  X is an arginine moiety in teh D-configuration


<220>
<221>  MISC_FEATURE
<222>  (4)..(4)
<223>  X is a 4-hydroxyproline moiety


<220>
<221>  MISC_FEATURE
<222>  (6)..(6)
<223>  X is a cyclopentylglycy moiety


<220>
<221>  MISC_FEATURE
<222>  (8)..(8)
<223>  X is a cyclopentylglycyl moiety in the D-configuration


<220>
<221>  MISC_FEATURE

<222>  (9)..(9)
<223>  X is a cyclopentylglycyl moiety


<400>  13

Xaa Arg Pro Xaa Gly Xaa Ser Xaa Xaa Arg
1               5                   10


<210>  14
<211>  10
<212>  PRT
<213>  Artificial

<220>
<221>  MISC_FEATURE
<222>  (1)..(1)
<223>  X is an arginine moiety in the D-configuratino


<220>
<221>  MISC_FEATURE
<222>  (6)..(6)
<223>  X is a 2-thienylalanyl moiety


<220>
<221>  MISC_FEATURE
<222>  (8)..(8)
<223>  X is a 1,2,3,4-tetrahydroisoquinoline-3-yl carbonyl moiety in the
       D-configuration


<220>
<221>  MISC_FEATURE
<222>  (9)..(9)
<223>  X is a cis, endo-octahydroindole-2-carbonyl moiety


<400>  14

Xaa Arg Pro Pro Gly Xaa Ser Xaa Xaa Arg
1               5                   10


<210>  15
<211>  9
<212>  PRT
<213>  Artificial

<220>
<221>  MISC_FEATURE
<222>  (1)..(1)
<223>  X is an arginine analog substituted with a tosyl group


<220>
<221>  MISC_FEATURE
<222>  (3)..(3)
<223>  X is a 4-hydroxyproline moiety

<220>
<221>  MISC_FEATURE
<222>  (5)..(5)
<223>  X is a 2-thienylalanyl moiety


<220>
<221>  MISC_FEATURE
<222>  (7)..(7)
<223>  X is a 1,2,3,4-tetrahydroisoquinoline-3-yl carbonyl moiety in teh
       D-configuration


<220>
<221>  MISC_FEATURE
<222>  (8)..(8)
<223>  X is a cis, endo-octahydroindole-2-carbonyl moiety


<400>  15

Xaa Pro Xaa Gly Xaa Ser Xaa Xaa Arg
1               5


<210>  16
<211>  9
<212>  PRT
<213>  Artificial

<220>
<221>  MISC_FEATURE
<222>  (1)..(1)
<223>  X is ab arginine analog substituted with a tosyl group


<220>
<221>  MISC_FEATURE
<222>  (3)..(3)
<223>  X is a 4-Hydroxyproline moiety


<220>
<221>  MISC_FEATURE
<222>  (7)..(7)
<223>  X is a 1,2,3,4-tetrahydroisoquinoline-3-yl carbonyl moiety in teh
       D-configuration


<220>
<221>  MISC_FEATURE
<222>  (8)..(8)
<223>  X is a cis, endo-octahydroindole-2-carbonyl moiety


<400>  16

Xaa Pro Xaa Gly Phe Ser Xaa Xaa Arg
1               5


<210>  17
<211>  10

<212>  PRT
<213>  Artificial

<220>
<221>  MISC_FEATURE
<222>  (1)..(1)
<223>  X is an arginine analog in the D- configuration


<220>
<221>  MISC_FEATURE
<222>  (4)..(4)
<223>  X is a 4-hydroxyproline moiety


<220>
<221>  MISC_FEATURE
<222>  (8)..(8)
<223>  X is a 1,2,3,4-tetrahydroisoquinoline-3-yl carbonyl moiety in the
       D configuration


<220>
<221>  MISC_FEATURE
<222>  (9)..(9)
<223>  X is cis, endo-octahydroindole-2-carbonyl moiety


<400>  17

Xaa Arg Pro Xaa Gly Phe Ser Xaa Xaa Arg
1               5                   10


<210>  18
<211>  10
<212>  PRT
<213>  Artificial

<220>
<221>  MISC_FEATURE
<222>  (1)..(1)
<223>  X is an arginine moiety which is in the D-configuration and is FM
      OC-protected


<220>
<221>  MISC_FEATURE
<222>  (4)..(4)
<223>  X is a 4-hydroxyproline moiety


<220>
<221>  MISC_FEATURE
<222>  (6)..(6)
<223>  X is a 2-thienylalanyl moiety


<220>
<221>  MISC_FEATURE
<222>  (8)..(8)
<223>  X is a 1,2,3,4-tetrahydroisoquinoline-3-yl carbonyl moiety in the
       D-configuration

<220>
<221>  MISC_FEATURE
<222>  (9)..(9)
<223>  X is a cis, endo-octahydroindole-2-carbonyl moiety


<400>  18

Xaa Arg Pro Xaa Gly Xaa Ser Xaa Xaa Arg
1               5                   10


<210>  19
<211>  11
<212>  PRT
<213>  Artificial

<220>
<221>  MISC_FEATURE
<222>  (1)..(1)
<223>  X is a cis, endo-2-azabicyclo[3.3.0]octane-3-S-carbonyl N-protect
       ed with a FMOC group


<220>
<221>  MISC_FEATURE
<222>  (2)..(2)
<223>  X is an arginine moiety in the D-configuration


<220>
<221>  MISC_FEATURE
<222>  (5)..(5)
<223>  X is a 4-hydroxyproline moiety


<220>
<221>  MISC_FEATURE
<222>  (7)..(7)
<223>  X is a 2-thienylalanyl moiety


<220>
<221>  MISC_FEATURE
<222>  (9)..(9)
<223>  X is a 1,2,3,4- tetrahydroisoquinoline-3-yl carbonyl moiety in th
       e D-configuration


<220>
<221>  MISC_FEATURE
<222>  (10)..(10)
<223>  X is a cis, endo-octahydroindole-2-carbonyl moiety


<400>  19

Xaa Xaa Arg Pro Xaa Gly Xaa Ser Xaa Xaa Arg
1               5                   10

<210>  20
<211>  11
<212>  PRT
<213>  Artificial

<220>
<221>  MISC_FEATURE
<222>  (1)..(1)
<223>  X is FMOC-epsilon-aminocaproyl

<220>
<221>  MISC_FEATURE
<222>  (2)..(2)
<223>  X is an arginine moiety in the D-configuration

<220>
<221>  MISC_FEATURE
<222>  (5)..(5)
<223>  X is a 2-Thienylalanyl moiety

<220>
<221>  MISC_FEATURE
<222>  (5)..(5)
<223>  X is a 4-hydroxyproline moiety

<220>
<221>  MISC_FEATURE
<222>  (7)..(7)
<223>  X is a 2-thienylalanyl moiety

<220>
<221>  MISC_FEATURE
<222>  (9)..(9)
<223>  X is a 1,2,3,4- tetrahydroisoquinoline-3-yl carbonyl moiety in th
       e D-configuration

<220>
<221>  MISC_FEATURE
<222>  (10)..(10)
<223>  X is a cis, endo-octahydroindole-2-carbonyl moiety

<400>  20

Xaa Xaa Arg Pro Xaa Gly Xaa Ser Xaa Xaa Arg
1               5                   10

<210>  21
<211>  10
<212>  PRT
<213>  Artificial

<220>
<221>  MISC_FEATURE
<222>  (1)..(1)
<223>  X is an arginine analog in the D-configuration N-substituted with

benzoyl

<220>
<221> MISC_FEATURE
<222> (4)..(4)
<223> X is a 4-hydroxyproline moiety

<220>
<221> MISC_FEATURE
<222> (6)..(6)
<223> X is a 2-thienylalanyl moiety

<220>
<221> MISC_FEATURE
<222> (8)..(8)
<223> X is a 1,2,3,4-tetrahydroisoquinoline-3-yl carbonyl moiety in the
 D-configuration

<220>
<221> MISC_FEATURE
<222> (9)..(9)
<223> X is a cis, endo-octahydroindole-2-carbonyl moiety

<400> 21

Xaa Arg Pro Xaa Gly Xaa Ser Xaa Xaa Arg
1               5                   10

<210> 22
<211> 10
<212> PRT
<213> Artificial

<220>
<221> MISC_FEATURE
<222> (1)..(1)
<223> X is an arginine in the D-configuration N-substituted with cycloh
 exylcarbonyl

<220>
<221> MISC_FEATURE
<222> (4)..(4)
<223> X is a 4-hydroxyproline moiety

<220>
<221> MISC_FEATURE
<222> (6)..(6)
<223> X is a thienylalanyl moiety

<220>
<221> MISC_FEATURE
<222> (8)..(8)
<223> X is a 1,2,3,4- tetrahydroisoquinoline-3-yl- carbonyl moiety in t
 he D-configuration

<220>
<221>  MISC_FEATURE
<222>  (9)..(9)
<223>  X is a cis, endo-octahydroindole-2-carbonyl moiety


<400>  22

Xaa Arg Pro Xaa Gly Xaa Ser Xaa Xaa Arg
1               5                   10


<210>  23
<211>  11
<212>  PRT
<213>  Artificial

<220>
<221>  MISC_FEATURE
<222>  (1)..(1)
<223>  X is a FMOC-N-(2-FMOC) aminoethyl)glycine moiety


<220>
<221>  MISC_FEATURE
<222>  (2)..(2)
<223>  X is an arginine moiety in the D-configuration


<220>
<221>  MISC_FEATURE
<222>  (5)..(5)
<223>  X is a 4-hydroxyproline moiety


<220>
<221>  MISC_FEATURE
<222>  (7)..(7)
<223>  X is a thienylalanyl moiety


<220>
<221>  MISC_FEATURE
<222>  (9)..(9)
<223>  X is a 1,2,3,4-tetrahydroisoquinoline-3-yl carbonyl moiety in the
       D-configuration


<220>
<221>  MISC_FEATURE
<222>  (10)..(10)
<223>  X is a cis, enod- octahydroindole-2-carbonyl moiety


<400>  23

Xaa Xaa Arg Pro Xaa Gly Xaa Ser Xaa Xaa Arg
1               5                   10


<210>  24

<211>  10
<212>  PRT
<213>  Artificial

<220>
<221>  MISC_FEATURE
<222>  (1)..(1)
<223>  X is a FMOC-N-(2-(FMOC)aminoethyl)glycine moiety


<220>
<221>  MISC_FEATURE
<222>  (4)..(4)
<223>  X is a 4-hydroxyproline moiety


<220>
<221>  MISC_FEATURE
<222>  (6)..(6)
<223>  X is a thienylalanine moiety


<220>
<221>  MISC_FEATURE
<222>  (8)..(8)
<223>  X is a 1,2,3,4-tetrahydroisoquinoline-3-yl carbonyl moiety in the
       D-configuration


<220>
<221>  MISC_FEATURE
<222>  (9)..(9)
<223>  X is a cis, endo-octahydroindole-2-carbonyl moiety


<400>  24

Xaa Arg Pro Xaa Gly Xaa Ser Xaa Xaa Arg
1               5                   10


<210>  25
<211>  10
<212>  PRT
<213>  Artificial

<220>
<221>  MISC_FEATURE
<222>  (1)..(1)
<223>  X is an arginine in teh D-configuration N-substituted with indol-
      3-yl acetyl


<220>
<221>  MISC_FEATURE
<222>  (4)..(4)
<223>  X is a 4-hydroxyproline moiety


<220>
<221>  MISC_FEATURE
<222>  (6)..(6)
<223>  X is a 4-thienylalanine moiety

<220>
<221>  MISC_FEATURE
<222>  (8)..(8)
<223>  X is a 1,2,3,4-tetrahydroisoquinoline-3yl carbonyl moiety in the
       D-configuration


<220>
<221>  MISC_FEATURE
<222>  (9)..(9)
<223>  X is a cis, endo-octahydroindole-2-carbonyl moiety


<400>  25

Xaa Arg Pro Xaa Gly Xaa Ser Xaa Xaa Arg
1               5                   10


<210>  26
<211>  10
<212>  PRT
<213>  Artificial

<220>
<221>  MISC_FEATURE
<222>  (1)..(1)
<223>  X is an arginine moiety in teh D-configuratoin N-substituted with
        dibenzylacetyl


<220>
<221>  MISC_FEATURE
<222>  (4)..(4)
<223>  X is a 4-hydroxyproline moiety


<220>
<221>  MISC_FEATURE
<222>  (6)..(6)
<223>  X is a 4-thienylalanine moiety


<220>
<221>  MISC_FEATURE
<222>  (8)..(8)
<223>  X is a 1,2,3,4-tetrahydroisoquinoline-3-yl carbonyl moiety in the
        D configuration


<220>
<221>  MISC_FEATURE
<222>  (9)..(9)
<223>  X is a cis, endo-octahydroindole-2-carbonyl moiety


<400>  26

Xaa Arg Pro Xaa Gly Xaa Ser Xaa Xaa Arg
1               5                   10